(19) Europäisches Patentamt
European Patent Office
Office européen des brevets

(11) EP 4 259 102 B1

(12) **EUROPEAN PATENT SPECIFICATION**

(45) Date of publication and mention of the grant of the patent:
**11.03.2026 Bulletin 2026/11**

(21) Application number: **21904299.1**

(22) Date of filing: **08.12.2021**

(51) International Patent Classification (IPC):
***A61K 9/06*** (2006.01) ***A61K 9/14*** (2006.01)
***A61K 31/16*** (2006.01) ***A61K 31/337*** (2006.01)
***A61K 31/78*** (2006.01) ***A61K 47/10*** (2017.01)
***A61K 47/56*** (2017.01) ***A61K 47/69*** (2017.01)
***A61K 47/54*** (2017.01) ***A61K 49/00*** (2006.01)
***A61P 27/02*** (2006.01) ***A61P 35/00*** (2006.01)
***A61K 31/496*** (2006.01) ***A61K 31/56*** (2006.01)
***A61K 31/573*** (2006.01) ***A61K 47/32*** (2006.01)

(52) Cooperative Patent Classification (CPC):
**A61K 31/56; A61K 9/0048; A61K 9/1075; A61K 9/5138; A61K 31/496; A61K 31/573; A61K 47/32; A61P 25/00; A61P 27/02; A61P 29/00; A61P 31/00; A61P 35/00**

(86) International application number:
**PCT/US2021/062324**

(87) International publication number:
**WO 2022/125611 (16.06.2022 Gazette 2022/24)**

(54) **COMPOSITIONS AND METHODS FOR MUCOSAL DRUG DELIVERY**

ZUSAMMENSETZUNGEN UND VERFAHREN ZUR MUKOSALEN ARZNEIMITTELABGABE

COMPOSITIONS ET MÉTHODES D'ADMINISTRATION DE MÉDICAMENT PAR VOIE MUQUEUSE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **09.12.2020 US 202063123208 P**

(43) Date of publication of application:
**18.10.2023 Bulletin 2023/42**

(73) Proprietors:
- **The Regents of The University of California Oakland CA 94607-5200 (US)**
- **Massachusetts Eye & Ear Infirmary Boston, MA 02114 (US)**
- **The Schepens Eye Research Institute, Inc. Boston, MA 02114 (US)**

(72) Inventors:
- **ANNABI, Nasim Los Angeles, CA 90095-7191 (US)**
- **DANA, Reza Los Angeles, CA 90095-7191 (US)**
- **GHOLIZADEH, Shima Los Angeles, CA 90095-7191 (US)**

(74) Representative: **Vossius & Partner Patentanwälte Rechtsanwälte mbB Siebertstraße 3 81675 München (DE)**

(56) References cited:
**WO-A1-01/87345**
**WO-A1-2013/188979**
**WO-A1-2016/195394**

- **TALELLI M ET AL: "Micelles based on HPMA copolymers", ADVANCED DRUG DELIVERY REVIEWS, ELSEVIER, AMSTERDAM , NL, vol. 62, no. 2, 17 February 2010 (2010-02-17), pages 231 - 239, XP026893655, ISSN: 0169-409X, [retrieved on 20100207], DOI: 10.1016/J.ADDR.2009.11.029**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- JUMELLE CLOTILDE ET AL: "Advances and limitations of drug delivery systems formulated as eye drops", JOURNAL OF CONTROLLED RELEASE, ELSEVIER, AMSTERDAM, NL, vol. 321, 3 February 2020 (2020-02-03), pages 1 - 22, XP086135610, ISSN: 0168-3659, [retrieved on 20200203], DOI: 10.1016/J.JCONREL.2020.01.057
- ZHANG X., JACKSON J.K. ET AL.: "Development of amphiphilic diblock copolymers as micellar carriers of taxol", INTERNATIONAL JOURNAL OF PHARMACEUTICS, ELSEVIER, NL, vol. 132, 1 January 1996 (1996-01-01), NL , pages 195 - 206, XP002213203, ISSN: 0378-5173, DOI: 10.1016/0378-5173(95)04386-1

## Description

### RELATED APPLICATIONS

This application claims the benefit of U.S. Provisional Application No. 63/123,208, filed December 9, 2020.

### STATEMENT OF GOVERNMENT SUPPORT

This invention was made with government support under Grant Number W81XWH-18-1-0654, awarded by the Department of Defense. The government has certain rights in the invention.

### BACKGROUND

Topical administration of drugs, including to the eye, currently remains a widely accepted administration route for the treatment of tissue pathologies including the front (anterior segment) of the eye. Topical application of drug is generally convenient and low cost with limited side effects due to lack of extensive systemic circulation of drug. The conventional ophthalmic medications, including eye drops, gels, suspensions and ointments, suffer mainly from poor ocular bioavailability (< 5%) after instillation.

Static barriers (including tight junctions and the conjunctiva, the alternating hydrophobic corneal epithelium and hydrophilic corneal stroma) and dynamic barriers (including the rapid tear turnover, vasculature and lymphatics of the conjunctiva), all contribute to the highly impenetrable anterior surface. Patent document WO 2013/188979 A1 discloses mucoadhesive delivery systems comprising nanoparticles.

Conventional formulations rely on frequent instillations of eye drops to maintain the therapeutic dose at the site of action. The main drawbacks of such therapies are: 1) possibly induced toxic side effects due to highly concentrated drug and cellular damages at the surface of cornea, and 2) noncompliance due to high frequency of application (which is one of the main reasons for deceased therapeutic efficiency). There is an unmet clinical need to develop additional formulations for enhanced drug delivery to the front of the eye.

### SUMMARY OF THE INVENTION

In one aspect, provided are pharmaceutical formulations, comprising:

a nanoparticle comprising an amphiphilic block copolymer according to the claims; and
an aqueous matrix comprising an anionic polymer;
wherein the nanoparticle is dispersed in the aqueous matrix.

### BRIEF DESCRIPTION OF THE DRAWINGS

**FIG. 1** is a schematic illustration of drug-loaded particles (i.e. micelles), surface modified with targeting moieties to form stable (covalent bonds) with trans-membrane mucin of epithelial cells. The final product will be in the form of MTPs embedded inside a matrix solution, able to release a variety of drugs.

**FIGs. 2A-2B** show the building blocks of Mucus targeting particles (MTPs). **FIG. 2A** is a schematic representation of micellar forming unit and drug loaded self-assembled (highly biocompatible) micelle. **FIG. 2B** is the molecular structure of block co-polymer designed for sustained release of hydrophobic drug molecules.

**FIGs. 3A-3C** show the characteristics of LE loaded micellar formulation. **FIG. 3A** shows the physicochemical characteristics of LE loaded micellar formulation formed at 10:2 polymer/drug ratio. **FIG. 3B** is the release profile of LE from micelles. Data points are fitted using logarithmic regression (solid blue line). **FIG. 3C** shows the concentration LE payload in micellar formulation using different polymer/drug ratio. Data are presented as mean values of 3 independent formulations ± SD.

**FIGs. 4A-4B** depict surface functionalization of micelles. **FIG. 4A** is a schematic representation of micellar surface modification either with phenylboronic acid which leads to boronate ester bond formation with Sialic acid units of adherent mucins via diol exchange reactions or surface modification with maleimide in order to form stable thiosuccinimide bond formation with adherent mucins on the surface of cornea. **FIG. 4B** is a $^1$HNMR spectra of synthesized $(Boc\text{-}NH\text{-}PEG)_2$-ABCPA macroinitiator using 400 MHz spectrometer.

**FIG. 5** is a schematic representation of a flow-through *ex-vivo* retention experiment in order to evaluate mucoadhesive properties and kinetics of fluorescein labeled targeting particles (i.e. micelles).

**FIG. 6** shows viscosity of matrix solution at 1.5 %w/v polycarbophil concentration as function of applied shear stress. Data was obtained using a controlled-stress rheometer.

**FIGs. 7A-7B** shows the experimental and control group for establishing non-inferiority of the antibiotic loaded MTPs formulation compared to commercialized product in **(FIG. 7A)** ex vivo and **(FIG. 7B)** in vivo models.

**FIG. 8** shows the experimental and control group for establishing non-inferiority of the anti-inflammatory loaded MTPs formulation compared to commercialized product in vivo.

**FIG. 9** shows the synthesis route of (PBA/Mal-PEG)2 -ABCPA.

**FIG. 10** shows the GPC chromatograms (RI signal) of (PEG)2 -ABCPA and $PEG_{5000}$.

**FIG. 11** shows the GPC chromatograms (RI signal) of (Boc-NH-PEG)2 -ABCPA.

**FIG. 12** shows the $^1$NMR spectrum of (Boc-PEG)2 -ABCPA in Chloroform.

**FIG. 13** shows the synthesis route of (targeted) PAB/Mal-PEG-b-p(HPMAm-$Lac_n$).

**FIG. 14** shows the synthesis route of (stealth) PEG-b-p(HPMAm-$Lac_n$).

**FIG. 15** shows the $^1$NMR spectrum of HPMA-$Lac_{2-4}$ in Chloroform.

**FIG. 16** shows the GPC traces (RI signal) of PEG-b-p(HPMAm-$Lac_{2-4}$) and $PEG_{5000}$.

**FIG. 17** shows the $^1$H NMR spectrum of PEG-b-p(HPMAm-$Lac_{2-4}$) in Chloroform.

## DETAILED DESCRIPTION OF THE INVENTION

This disclosure provides epithelial cell-trans-membrane mucin targeting eye drops with sustained drug release profile to manage inflammation and prevent infection after military-related ocular injuries. More specifically, disclosed is related to development of an ocular drug delivery in a sustained manner in order to improve the drugs' therapeutic efficacy and decrease the frequency of eye drop installation.

To address the need of providing anti-inflammatory and/or anti-bacterial therapies for ocular inflammation/infection management, the present disclosure describes, in certain embodiments, a new drug delivery platform. As fully set forth below, the formulations described herein provide high surface and retention penetration (e.g., on the eye or other mucous membranes) for sustained drug delivery purposes, which will lead to increased therapeutic efficacy and decreased frequency of application **(FIG. 1).** This topical platform will significantly decrease the frequency of application from 3-4 (or more) times a day down to once daily or more preferably to once every 2-3 days.

In one aspect, provided herein are pharmaceutical formulations, comprising:

a nanoparticle comprising an amphiphilic block copolymer according to the claims; and
an aqueous matrix comprising an anionic polymer;
wherein the nanoparticle is dispersed in the aqueous matrix.

In certain embodiments, the nanoparticle is a micelle.

In certain embodiments, the amphiphilic block copolymer is an amphiphilic diblock copolymer, comprising a hydrophobic block and a hydrophilic block.

In certain embodiments, the amphiphilic block copolymer is an amphiphilic triblock copolymer, comprising two hydrophobic blocks and a hydrophilic block, wherein the two hydrophobic blocks are separated by the hydrophilic block.

In certain embodiments, the amphiphilic block copolymer is an amphiphilic triblock copolymer, comprising two hydrophilic blocks and a hydrophobic block, wherein the two hydrophilic blocks are separated by the hydrophobic block.

According to the claims, the hydrophilic block of the amphiphilic block copolymer comprises a repeat unit having the following structure:

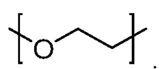

According to the claims, the hydrophilic block of the amphiphilic block copolymer is a poly(ethylene glycol) having the following structure:

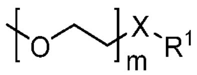

wherein X is O, NH, or S; $R^1$ is hydrogen, alkyl, alkenyl, alkynyl, carbocyclyl, heterocyclyl, aryl, or heteroaryl; and m is an integer from 5 to 2000.

In certain embodiments, the hydrophilic block has a number-average molecular weight of about 500 Da to about 20 kDa.

In certain embodiments, the hydrophilic block has a number-average molecular weight of about 1 kDa to about 8 kDa.

According to the claims, the hydrophobic block of the amphiphilic block copolymer comprises a repeat unit having the following structure:

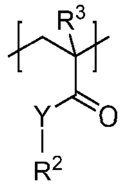

wherein:

Y is O, NH, or S;
$R^2$ is optionally substituted alkyl, alkenyl, alkynyl, carbocyclyl, heterocyclyl, aryl, or heteroaryl; and
$R^3$ is H or alkyl;.

According to the claims, $R^3$ is methyl and X is NH.

According to the claims, the hydrophobic block of the amphiphilic block copolymer comprises a repeat unit having the following structure:

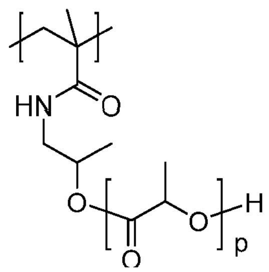

wherein p is integer from 1 to 10.

In certain embodiments, p is integer from 1 to 5. In certain embodiments, p is 4.

According to the claims, the hydrophobic block of the amphiphilic block copolymer has the following structure:

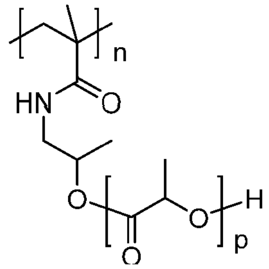

wherein n is an integer from 5 to 2000.

According to the claims, each p is an integer independently selected from 1 to 10. In certain preferred embodiments, each p is an integer independently selected from 1 to 5. In certain such embodiments, p is independently selected from 1, 2, 3, or 4. In certain such embodiments, at least 5% of the repeat units designated by n have p independently selected from 3 or 4. In certain such embodiments, about 80% of the repeat units designated by n have p of 1 or 2. In certain embodiments, 5-20% of the repeat units designated by n have p of 3 or 4 and 80-95% of the repeat units designated by n have p of 1 or 2.

In certain embodiments, the hydrophobic block has a number-average molecular weight of about 500 Da to about 200 kDa. In certain embodiments, the hydrophobic block has a number-average molecular weight of about 1 kDa to about 50 kDa. In certain embodiments, the hydrophobic block has a number-average molecular weight of about 2 kDa to about 20 kDa.

According to the claims, the amphiphilic block copolymer has the following structure:

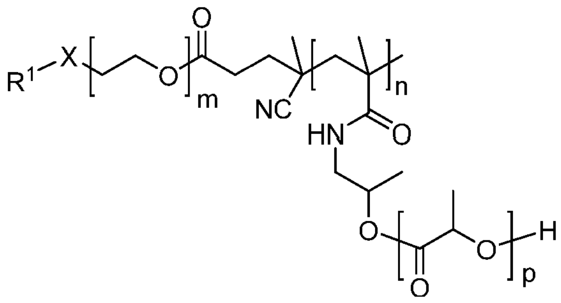

,

wherein X is O, NH, or S; $R^1$ is hydrogen, alkyl, alkenyl, alkynyl, carbocyclyl, heterocyclyl, aryl, or heteroaryl; m and n are each independently an integer from 5-2000; and p is an integer from 1 to 10.

According to the claims, each p is an integer independently selected from 1 to 10. In certain preferred embodiments, each p is an integer independently selected from 1 to 5. In certain such embodiments, p is independently selected from 1, 2, 3, or 4. In certain such embodiments, at least 5% of the repeat units designated by n have p independently selected from 3 or 4. In certain such embodiments, about 80% of the repeat units designated by n have p of 1 or 2. In certain embodiments, 5-20% of the repeat units designated by n have p of 3 or 4 and 80-95% of the repeat units designated by n have p of 1 or 2.

In certain embodiments, the nanoparticle has a diameter of about 10 nm to about 1000 nm. In certain embodiments, the nanoparticle has a diameter of about 30 nm to about 500 nm. In certain embodiments, the nanoparticle has a diameter of about 50 nm to about 250 nm.

In certain embodiments, the surface of the nanoparticle comprises a glycoprotein targeting moiety.

In certain embodiments, the glycoprotein targeting moiety comprises a boronic acid group:

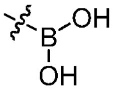

.

In certain embodiments, the glycoprotein targeting moiety has the following structure:

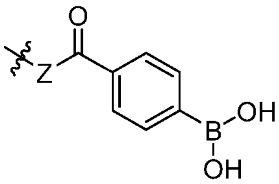

,

wherein Z is O, NH, or S.

In certain embodiments, the glycoprotein targeting moiety comprises a maleimide group:

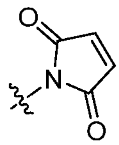

.

In certain embodiments, the glycoprotein targeting moiety has the following structure:

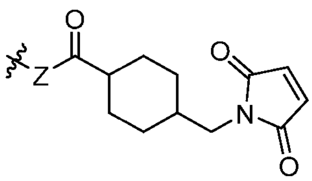

,

wherein Z is O, NH, or S.

In certain embodiments, the glycoprotein targeting moiety is coupled to the distal end (e.g., the amine, hydroxyl or thiol) of the hydrophilic block.

In certain embodiments, in the nanoparticle further comprises a therapeutic agent.

In certain embodiments, the therapeutic agent is an antibacterial agent or an anti-inflammatory agent.

In certain embodiments, the antibacterial agent or anti-inflammatory agent is selected from Loteprednol etabonate, dexamethasone, and Ciprofloxacin.

In certain embodiments, the anionic polymer is cross-linked, preferably wherein the cross-linked anionic polymer has a cross-linking density of from about 0.01% to about 2.0%.

In certain embodiments, the cross-linking density of the anionic polymer is from about 0.05% to about 0.5%.

In certain embodiments, the anionic polymer comprises a repeat unit selected from

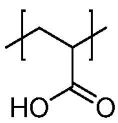 and 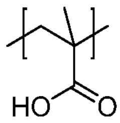.

In certain embodiments, the anionic polymer comprises a cross-linking unit derived from divinyl glycol.

In certain embodiments, the anionic polymer is polycarbophil.

In certain embodiments, the polycarbophil is calcium polycarbophil.

In certain embodiments, the matrix has a polycarbophil weight to volume percent (%w/v) of about 0.01% to about 10.0%. In certain embodiments, the matrix has a polycarbophil weight to volume percent (%w/v) of about 0.1% to about 2.0%.

In certain embodiments, the aqueous matrix comprising the cross-linked ionic polymer is a non-settling gel.

In certain embodiments, the matrix further comprises Tyloxapol.

In certain embodiments, the matrix has a Tyloxapol weight to volume percent (%w/v) of about 0.01% to about 5.0%. In certain embodiments, the matrix has a Tyloxapol weight to volume percent (%w/v) of about 0.1% to about 2.0%. In certain embodiments, he matrix has a Tyloxapol weight to volume percent (%w/v) of about 0.5%.

In certain embodiments, the aqueous matrix further comprises a chelating agent.

In certain embodiments, the chelating agent is edetate disodium dihydrate (EDTA).

In certain embodiments, the pharmaceutical formulation is formulated for topical administration.

In certain embodiments, the pharmaceutical formulation is formulated for topical administration as an ophthalmic drop.

In another aspect, provided herein are methods of administering a therapeutic agent, comprising administering the pharmaceutical formulation disclosed herein to an eye of a patient in need thereof.

In certain embodiments, the pharmaceutical formulation is administered to the anterior segment of the eye.

In yet another aspect, provided herein are amphiphilic diblock copolymers, comprising a hydrophilic block and a hydrophobic block,
wherein:

the hydrophilic block comprises a repeat unit having the following structure:

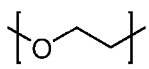,

and
the hydrophobic block comprises a repeat unit having the following structure:

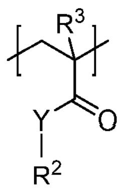

wherein:

Y is O, NH, or S;

$R^2$ is alkyl, alkenyl, alkynyl, carbocyclyl, heterocyclyl, aryl, or heteroaryl; and
$R^3$ is H or methyl;
the distal end of the hydrophilic block comprises a glycoprotein targeting moiety that comprises a boronic acid group, or a maleimide group.

In certain embodiments, the glycoprotein targeting moiety has the following structure:

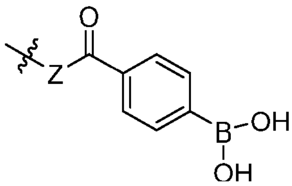

wherein Z is O, NH, or S.

In certain embodiments, the glycoprotein targeting moiety has the following structure:

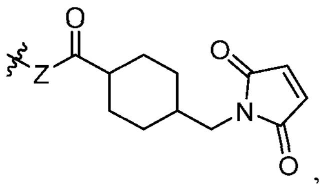

wherein Z is O, NH, or S.

According to the claims, the hydrophobic block comprises a repeat unit having the following structure:

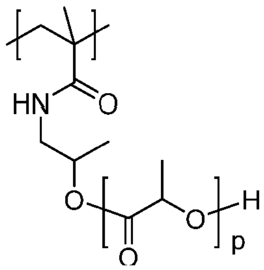

wherein p is an integer from 1 to 10.

According to the claims, the hydrophobic block has the following structure:

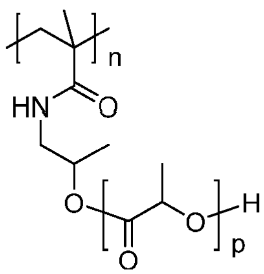

wherein n is an integer from 5 to 2000.

In certain embodiments, each p is an integer independently selected from 1 to 10. In certain preferred embodiments, each p is an integer independently selected from 1 to 5. In certain such embodiments, p is independently selected from 1, 2, 3, or 4. In certain such embodiments, at least 5% of the repeat units designated by n have p independently selected from 3 or 4. In certain such embodiments, about 80% of the repeat units designated by n have p of 1 or 2. In certain embodiments, 5-20% of the repeat units designated by n have p of 3 or 4 and 80-95% of the repeat units designated by n have p of 1 or 2.

According to the claims, the amphiphilic diblock copolymer has the following structure:

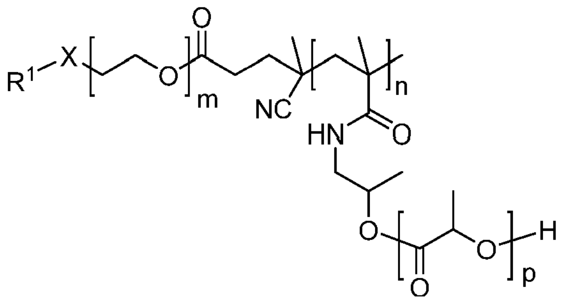

wherein X is O, NH, or S; $R^1$ is hydrogen, alkyl, alkenyl, alkynyl, carbocyclyl, heterocyclyl, aryl, or heteroaryl; m and n each independently is an integer from 5-2000; and p is an integer from 1 to 10.

According to the claims, each p is an integer independently selected from 1 to 10. In certain preferred embodiments, each p is an integer independently selected from 1 to 5. In certain such embodiments, p is independently selected from 1, 2, 3, or 4. In certain such embodiments, at least 5% of the repeat units designated by n have p independently selected from 3 or 4. In certain such embodiments, about 80% of the repeat units designated by n have p of 1 or 2. In certain embodiments, 5-20% of the repeat units designated by n have p of 3 or 4 and 80-95% of the repeat units designated by n have p of 1 or 2.

According to the claims, provided are nanoparticles, comprising the amphiphilic diblock copolymer disclosed herein.

In certain embodiments, the nanoparticle has an average diameter of about 10 nm to about 1000 nm.

In certain embodiments, the nanoparticle has an average diameter of about 30 nm to about 500 nm.

In certain embodiments, the nanoparticle has an average diameter of about 50 nm to about 250 nm.

In certain embodiments, the nanoparticle further comprises a therapeutic agent.

In certain embodiments, the therapeutic agent is selected from Loteprednol etabonate, dexamethasone, and Ciprofloxacin.

Pharmaceutical Composition

The compositions of the present invention may be utilized to treat an individual in need thereof (not claimed). In certain embodiments, the individual is a mammal such as a human, or a non-human mammal. When administered to an animal, such as a human, the composition, the compound, or the nanoparticle is preferably administered as a pharmaceutical composition comprising, for example, a compound or nanoparticle of the invention and a pharmaceutically acceptable carrier. Pharmaceutically acceptable carriers are well known in the art and include, for example, aqueous solutions such as water or physiologically buffered saline or other solvents or vehicles such as glycols, glycerol, oils such as olive oil, or injectable organic esters. In preferred embodiments, when such pharmaceutical compositions are for human administration, particularly for invasive routes of administration (i.e., routes, such as injection or implantation, that circumvent transport or diffusion through an epithelial barrier), the aqueous solution is pyrogen-free, or substantially pyrogen-free. The excipients can be chosen, for example, to effect delayed release of an agent or to selectively target one or more cells, tissues or organs. The pharmaceutical composition can be in dosage unit form such as tablet, capsule (including sprinkle capsule and gelatin capsule), granule, lyophile for reconstitution, powder, solution, syrup, suppository, injection or the like. The composition can also be present in a transdermal delivery system, e.g., a skin patch. The composition can also be present in a solution suitable for topical administration, such as a lotion, cream, or ointment.

A pharmaceutically acceptable carrier can contain physiologically acceptable agents that act, for example, to stabilize, increase solubility or to increase the absorption of a compound or nanoparticle such as a compound or nanoparticle of the invention. Such physiologically acceptable agents include, for example, carbohydrates, such as glucose, sucrose or dextrans, antioxidants, such as ascorbic acid or glutathione, chelating agents, low molecular weight proteins or other stabilizers or excipients. The choice of a pharmaceutically acceptable carrier, including a physiologically acceptable agent, depends, for example, on the route of administration of the composition. The preparation or pharmaceutical composition can be a selfemulsifying drug delivery system or a selfmicroemulsifying drug delivery system. The pharmaceutical composition (preparation) also can be a liposome or other polymer matrix, which can have incorporated therein, for example, a compound or nanoparticle of the invention. Liposomes, for example, which comprise phospholipids or other lipids, are nontoxic, physiologically acceptable and metabolizable carriers that are relatively simple to make and administer.

The phrase "pharmaceutically acceptable" is employed herein to refer to those compounds or nanoparticles, materials, compositions, and/or dosage forms which are, within the scope of sound medical judgment, suitable for use in contact with the tissues of human beings and animals without excessive toxicity, irritation, allergic response, or other

problem or complication, commensurate with a reasonable benefit/risk ratio.

The phrase "pharmaceutically acceptable carrier" as used herein means a pharmaceutically acceptable material, composition or vehicle, such as a liquid or solid filler, diluent, excipient, solvent or encapsulating material. Each carrier must be "acceptable" in the sense of being compatible with the other ingredients of the formulation and not injurious to the patient. Some examples of materials which can serve as pharmaceutically acceptable carriers include: (1) sugars, such as lactose, glucose and sucrose; (2) starches, such as corn starch and potato starch; (3) cellulose, and its derivatives, such as sodium carboxymethyl cellulose, ethyl cellulose and cellulose acetate; (4) powdered tragacanth; (5) malt; (6) gelatin; (7) talc; (8) excipients, such as cocoa butter and suppository waxes; (9) oils, such as peanut oil, cottonseed oil, safflower oil, sesame oil, olive oil, corn oil and soybean oil; (10) glycols, such as propylene glycol; (11) polyols, such as glycerin, sorbitol, mannitol and polyethylene glycol; (12) esters, such as ethyl oleate and ethyl laurate; (13) agar; (14) buffering agents, such as magnesium hydroxide and aluminum hydroxide; (15) alginic acid; (16) pyrogen-free water; (17) isotonic saline; (18) Ringer's solution; (19) ethyl alcohol; (20) phosphate buffer solutions; and (21) other non-toxic compatible substances employed in pharmaceutical formulations.

A pharmaceutical composition (preparation) can be administered to a subject by any of a number of routes of administration including, for example, orally (for example, drenches as in aqueous or non-aqueous solutions or suspensions, tablets, capsules (including sprinkle capsules and gelatin capsules), boluses, powders, granules, pastes for application to the tongue); absorption through the oral mucosa (e.g., sublingually); subcutaneously; transdermally (for example as a patch applied to the skin); and topically (for example, as a cream, ointment or spray applied to the skin). The compound or nanoparticle may also be formulated for inhalation. **In** certain embodiments, a compound or nanoparticle may be simply dissolved or suspended in sterile water. Details of appropriate routes of administration and compositions suitable for same can be found in, for example, U.S. Pat. Nos. 6,110,973, 5,763,493, 5,731,000, 5,541,231, 5,427,798, 5,358,970 and 4,172,896, as well as in patents cited therein.

The formulations may conveniently be presented in unit dosage form and may be prepared by any methods well known in the art of pharmacy. The amount of active ingredient which can be combined with a carrier material to produce a single dosage form will vary depending upon the host being treated, the particular mode of administration. The amount of active ingredient that can be combined with a carrier material to produce a single dosage form will generally be that amount of the compound or nanoparticle which produces a therapeutic effect. Generally, out of one hundred percent, this amount will range from about 1 percent to about ninety-nine percent of active ingredient, preferably from about 5 percent to about 70 percent, most preferably from about 10 percent to about 30 percent.

Methods of preparing these formulations or compositions include the step of bringing into association an active compound or nanoparticle of the invention, with the carrier and, optionally, one or more accessory ingredients. In general, the formulations are prepared by uniformly and intimately bringing into association a compound or nanoparticle of the present invention with liquid carriers, or finely divided solid carriers, or both, and then, if necessary, shaping the product.

Suspensions, in addition to the active compounds or nanoparticles may contain suspending agents as, for example, ethoxylated isostearyl alcohols, polyoxyethylene sorbitol and sorbitan esters, microcrystalline cellulose, aluminum metahydroxide, bentonite, agar-agar and tragacanth, and mixtures thereof.

Dosage forms for the topical or transdermal administration include powders, sprays, ointments, pastes, creams, lotions, gels, solutions, patches and inhalants. The active compound or nanoparticle may be mixed under sterile conditions with a pharmaceutically acceptable carrier, and with any preservatives, buffers, or propellants that may be required.

The ointments, pastes, creams and gels may contain, in addition to an active compound or nanoparticle, excipients, such as animal and vegetable fats, oils, waxes, paraffins, starch, tragacanth, cellulose derivatives, polyethylene glycols, silicones, bentonites, silicic acid, talc and zinc oxide, or mixtures thereof.

Powders and sprays can contain, in addition to an active compound or nanoparticle, excipients such as lactose, talc, silicic acid, aluminum hydroxide, calcium silicates and polyamide powder, or mixtures of these substances. Sprays can additionally contain customary propellants, such as chlorofluorohydrocarbons and volatile unsubstituted hydrocarbons, such as butane and propane.

Transdermal patches have the added advantage of providing controlled delivery of a compound or nanoparticle of the present invention to the body. Such dosage forms can be made by dissolving or dispersing the active compound or nanoparticle in the proper medium. Absorption enhancers can also be used to increase the flux of the compound or nanoparticle across the skin. The rate of such flux can be controlled by either providing a rate controlling membrane or dispersing the compound or nanoparticle in a polymer matrix or gel.

The phrases "parenteral administration" and "administered parenterally" as used herein means modes of administration other than enteral and topical administration, usually by injection, and includes, without limitation, intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal and intrasternal injection and infusion. Pharmaceutical compositions suitable for parenteral administration comprise one or more active compounds or nanoparticles in combination with one or more pharmaceutically acceptable sterile isotonic aqueous or nonaqueous solutions, dispersions, suspensions or emulsions, or sterile powders which may be reconstituted into sterile injectable

solutions or dispersions just prior to use, which may contain antioxidants, buffers, bacteriostats, solutes which render the formulation isotonic with the blood of the intended recipient or suspending or thickening agents.

Examples of suitable aqueous and nonaqueous carriers that may be employed in the pharmaceutical compositions of the invention include water, ethanol, polyols (such as glycerol, propylene glycol, polyethylene glycol, and the like), and suitable mixtures thereof, vegetable oils, such as olive oil, and injectable organic esters, such as ethyl oleate. Proper fluidity can be maintained, for example, by the use of coating materials, such as lecithin, by the maintenance of the required particle size in the case of dispersions, and by the use of surfactants.

These compositions may also contain adjuvants such as preservatives, wetting agents, emulsifying agents and dispersing agents. Prevention of the action of microorganisms may be ensured by the inclusion of various antibacterial and antifungal agents, for example, paraben, chlorobutanol, phenol sorbic acid, and the like. It may also be desirable to include isotonic agents, such as sugars, sodium chloride, and the like into the compositions. In addition, prolonged absorption of the injectable pharmaceutical form may be brought about by the inclusion of agents that delay absorption such as aluminum monostearate and gelatin.

In some cases, in order to prolong the effect of a drug, it is desirable to slow the absorption of the drug from subcutaneous or intramuscular injection. This may be accomplished by the use of a liquid suspension of crystalline or amorphous material having poor water solubility. The rate of absorption of the drug then depends upon its rate of dissolution, which, in turn, may depend upon crystal size and crystalline form. Alternatively, delayed absorption of a parenterally administered drug form is accomplished by dissolving or suspending the drug in an oil vehicle.

Injectable depot forms are made by forming microencapsulated matrices of the subject compounds or nanoparticles in biodegradable polymers such as polylactidepolyglycolide. Depending on the ratio of drug to polymer, and the nature of the particular polymer employed, the rate of drug release can be controlled. Examples of other biodegradable polymers include poly(orthoesters) and poly(anhydrides). Depot injectable formulations are also prepared by entrapping the drug in liposomes or microemulsions that are compatible with body tissue.

For use in the methods of this invention, active compounds or nanoparticles can be given per se or as a pharmaceutical composition containing, for example, 0.1 to 99.5% (more preferably, 0.5 to 90%) of active ingredient in combination with a pharmaceutically acceptable carrier.

Methods of introduction may also be provided by rechargeable or biodegradable devices. Various slow release polymeric devices have been developed and tested *in vivo* in recent years for the controlled delivery of drugs, including proteinaceous biopharmaceuticals. A variety of biocompatible polymers (including hydrogels), including both biodegradable and non-degradable polymers, can be used to form an implant for the sustained release of a compound or nanoparticle at a particular target site.

Actual dosage levels of the active ingredients in the pharmaceutical compositions may be varied so as to obtain an amount of the active ingredient that is effective to achieve the desired therapeutic response for a particular patient, composition, and mode of administration, without being toxic to the patient.

The selected dosage level will depend upon a variety of factors including the activity of the particular compound or nanoparticle or combination of compounds or nanoparticles employed, or the ester, salt or amide thereof, the route of administration, the time of administration, the rate of excretion of the particular compound(s) or nanoparticle(s) being employed, the duration of the treatment, other drugs, compounds, nanoparticles, and/or materials used in combination with the particular compound(s) or nanoparticle(s) employed, the age, sex, weight, condition, general health and prior medical history of the patient being treated, and like factors well known in the medical arts.

A physician or veterinarian having ordinary skill in the art can readily determine and prescribe the therapeutically effective amount of the pharmaceutical composition required. For example, the physician or veterinarian could start doses of the pharmaceutical composition or compound or nanoparticle at levels lower than that required in order to achieve the desired therapeutic effect and gradually increase the dosage until the desired effect is achieved. By "therapeutically effective amount" is meant the concentration of a compound or nanoparticle that is sufficient to elicit the desired therapeutic effect. It is generally understood that the effective amount of the compound or nanoparticle will vary according to the weight, sex, age, and medical history of the subject. Other factors which influence the effective amount may include, but are not limited to, the severity of the patient's condition, the disorder being treated, the stability of the compound or nanoparticle, and, if desired, another type of therapeutic agent being administered with the compound or nanoparticle of the invention. A larger total dose can be delivered by multiple administrations of the agent. Methods to determine efficacy and dosage are known to those skilled in the art (Isselbacher et al. (1996) Harrison's Principles of Internal Medicine 13 ed., 1814-1882.

In general, a suitable daily dose of an active compound or nanoparticle used in the compositions and methods of the invention will be that amount of the compound or nanoparticle that is the lowest dose effective to produce a therapeutic effect. Such an effective dose will generally depend upon the factors described above.

If desired, the effective daily dose of the active compound or nanoparticle may be administered as one, two, three, four, five, six or more sub-doses administered separately at appropriate intervals throughout the day, optionally, in unit dosage forms. In certain embodiments of the present invention, the active compound or nanoparticle may be administered

two or three times daily. In preferred embodiments, the active compound or nanoparticle will be administered once daily.

The patient receiving this treatment is any animal in need, including primates, in particular humans; and other mammals such as equines, cattle, swine, sheep, cats, and dogs; poultry; and pets in general.

## *Definitions*

Unless otherwise defined herein, scientific and technical terms used in this application shall have the meanings that are commonly understood by those of ordinary skill in the art. Generally, nomenclature used in connection with, and techniques of, chemistry, cell and tissue culture, molecular biology, cell and cancer biology, neurobiology, neurochemistry, virology, immunology, microbiology, pharmacology, genetics and protein and nucleic acid chemistry, described herein, are those well known and commonly used in the art.

The methods and techniques of the present disclosure are generally performed, unless otherwise indicated, according to conventional methods well known in the art and as described in various general and more specific references that are cited and discussed throughout this specification. See, e.g., "Principles of Neural Science", McGraw-Hill Medical, New York, N.Y. (2000); Motulsky, "Intuitive Biostatistics", Oxford University Press, Inc. (1995); Lodish et al., "Molecular Cell Biology, 4th ed.", W. H. Freeman & Co., New York (2000); Griffiths et al., "Introduction to Genetic Analysis, 7th ed.", W. H. Freeman & Co., N.Y. (1999); and Gilbert et al., "Developmental Biology, 6th ed.", Sinauer Associates, Inc., Sunderland, MA (2000).

Chemistry terms used herein, unless otherwise defined herein, are used according to conventional usage in the art, as exemplified by "The McGraw-Hill Dictionary of Chemical Terms", Parker S., Ed., McGraw-Hill, San Francisco, C.A. (1985).

**In** case of conflict, the present specification, including its specific definitions, will control.

The term "agent" is used herein to denote a chemical compound or nanoparticle (such as an organic or inorganic compound or nanoparticle, a mixture of chemical compounds or nanoparticles, etc.), a biological macromolecule (such as a nucleic acid, an antibody, including parts thereof as well as humanized, chimeric and human antibodies and monoclonal antibodies, a protein or portion thereof, e.g., a peptide, a lipid, a carbohydrate), or an extract made from biological materials such as bacteria, plants, fungi, or animal (particularly mammalian) cells or tissues. Agents include, for example, agents whose structure is known, and those whose structure is not known. The ability of such agents to inhibit AR or promote AR degradation may render them suitable as "therapeutic agents" in the methods and compositions of this disclosure.

A "patient," "subject," or "individual" are used interchangeably and refer to either a human or a non-human animal. These terms include mammals, such as humans, primates, livestock animals (including bovines, porcines, etc.), companion animals (e.g., canines, felines, etc.) and rodents (e.g., mice and rats).

"Treating" a condition or patient refers to taking steps to obtain beneficial or desired results, including clinical results. As used herein, and as well understood in the art, "treatment" is an approach for obtaining beneficial or desired results, including clinical results. Beneficial or desired clinical results can include, but are not limited to, alleviation or amelioration of one or more symptoms or conditions, diminishment of extent of disease, stabilized (i.e. not worsening) state of disease, preventing spread of disease, delay or slowing of disease progression, amelioration or palliation of the disease state, and remission (whether partial or total), whether detectable or undetectable. "Treatment" can also mean prolonging survival as compared to expected survival if not receiving treatment.

The term "preventing" is art-recognized, and when used in relation to a condition, such as a local recurrence (e.g., pain), a disease such as cancer, a syndrome complex such as heart failure or any other medical condition, is well understood in the art, and includes administration of a composition which reduces the frequency of, or delays the onset of, symptoms of a medical condition in a subject relative to a subject which does not receive the composition. Thus, prevention of cancer includes, for example, reducing the number of detectable cancerous growths in a population of patients receiving a prophylactic treatment relative to an untreated control population, and/or delaying the appearance of detectable cancerous growths in a treated population versus an untreated control population, e.g., by a statistically and/or clinically significant amount.

"Administering" or "administration of" a substance, a compound, nanoparticle or an agent to a subject can be carried out using one of a variety of methods known to those skilled in the art. For example, a compound, nanoparticle, or an agent can be administered, intravenously, arterially, intradermally, intramuscularly, intraperitoneally, subcutaneously, ocularly, sublingually, orally (by ingestion), intranasally (by inhalation), intraspinally, intracerebrally, and transdermally (by absorption, e.g., through a skin duct). A compound, nanoparticle, or agent can also appropriately be introduced by rechargeable or biodegradable polymeric devices or other devices, e.g., patches and pumps, or formulations, which provide for the extended, slow or controlled release of the compound, nanoparticle, or agent. Administering can also be performed, for example, once, a plurality of times, and/or over one or more extended periods.

Appropriate methods of administering a substance, a compound, a nanoparticle, or an agent to a subject will also depend, for example, on the age and/or the physical condition of the subject and the chemical and biological properties of the compound, nanoparticle, or agent (e.g., solubility, digestibility, bioavailability, stability and toxicity). **In** some embodiments, a compound, nanoparticle, or an agent is administered orally, e.g., to a subject by ingestion. **In** some embodiments, the orally administered compound, nanoparticle, or agent is in an extended release or slow release formulation, or administered using a device for such slow or extended release.

A "therapeutically effective amount" or a "therapeutically effective dose" of a drug or agent is an amount of a drug or an agent that, when administered to a subject will have the intended therapeutic effect. The full therapeutic effect does not necessarily occur by administration of one dose, and may occur only after administration of a series of doses. Thus, a therapeutically effective amount may be administered in one or more administrations. The precise effective amount needed for a subject will depend upon, for example, the subject's size, health and age, and the nature and extent of the condition being treated, such as cancer or MDS. The skilled worker can readily determine the effective amount for a given situation by routine experimentation.

As used herein, the terms "optional" or "optionally" mean that the subsequently described event or circumstance may occur or may not occur, and that the description includes instances where the event or circumstance occurs as well as instances in which it does not. For example, "optionally substituted alkyl" refers to the alkyl may be substituted as well as where the alkyl is not substituted.

It is understood that substituents and substitution patterns on the compounds or nanoparticles of the present invention can be selected by one of ordinary skilled person in the art to result chemically stable compounds or nanoparticles which can be readily synthesized by techniques known in the art, as well as those methods set forth below, from readily available starting materials. If a substituent is itself substituted with more than one group, it is understood that these multiple groups may be on the same carbon or on different carbons, so long as a stable structure results.

As used herein, the term "optionally substituted" refers to the replacement of one to six hydrogen radicals in a given structure with the radical of a specified substituent including, but not limited to: hydroxyl, hydroxyalkyl, alkoxy, halogen, alkyl, nitro, silyl, acyl, acyloxy, aryl, cycloalkyl, heterocyclyl, amino, aminoalkyl, cyano, haloalkyl, haloalkoxy, $-OCO-CH_2-O-alkyl$, $-OP(O)(O-alkyl)_2$ or $-CH_2-OP(O)(O-alkyl)_2$. Preferably, "optionally substituted" refers to the replacement of one to four hydrogen radicals in a given structure with the substituents mentioned above. More preferably, one to three hydrogen radicals are replaced by the substituents as mentioned above. It is understood that the substituent can be further substituted.

As used herein, the term "alkyl" refers to saturated aliphatic groups, including but not limited to $C_1$-$C_{10}$ straight-chain alkyl groups or $C_1$-$C_{10}$ branched-chain alkyl groups. Preferably, the "alkyl" group refers to $C_1$-$C_6$ straight-chain alkyl groups or $C_1$-$C_6$ branched-chain alkyl groups. Most preferably, the "alkyl" group refers to $C_1$-$C_4$ straight-chain alkyl groups or $C_1$-$C_4$ branched-chain alkyl groups. Examples of "alkyl" include, but are not limited to, methyl, ethyl, 1-propyl, 2-propyl, n-butyl, sec-butyl, tert-butyl, 1-pentyl, 2-pentyl, 3-pentyl, neo-pentyl, 1-hexyl, 2-hexyl, 3-hexyl, 1-heptyl, 2-heptyl, 3-heptyl, 4-heptyl, 1-octyl, 2-octyl, 3-octyl or 4-octyl and the like. The "alkyl" group may be optionally substituted.

The term "acyl" is art-recognized and refers to a group represented by the general formula hydrocarbylC(O)-, preferably alkylC(O)-.

The term "acylamino" is art-recognized and refers to an amino group substituted with an acyl group and may be represented, for example, by the formula hydrocarbylC(O)NH-.

The term "acyloxy" is art-recognized and refers to a group represented by the general formula hydrocarbylC(O)O-, preferably alkylC(O)O-.

The term "alkoxy" refers to an alkyl group having an oxygen attached thereto. Representative alkoxy groups include methoxy, ethoxy, propoxy, tert-butoxy and the like.

The term "alkoxyalkyl" refers to an alkyl group substituted with an alkoxy group and may be represented by the general formula alkyl-O-alkyl.

The term "alkyl" refers to saturated aliphatic groups, including straight-chain alkyl groups, branched-chain alkyl groups, cycloalkyl (alicyclic) groups, alkyl-substituted cycloalkyl groups, and cycloalkyl-substituted alkyl groups. In preferred embodiments, a straight chain or branched chain alkyl has 30 or fewer carbon atoms in its backbone (e.g., $C_{1-30}$ for straight chains, $C_{3-30}$ for branched chains), and more preferably 20 or fewer.

Moreover, the term "alkyl" as used throughout the specification, examples, and claims is intended to include both unsubstituted and substituted alkyl groups, the latter of which refers to alkyl moieties having substituents replacing a hydrogen on one or more carbons of the hydrocarbon backbone, including haloalkyl groups such as trifluoromethyl and 2,2,2-trifluoroethyl, etc.

The term "$C_{x-y}$" or "$C_x$-$C_y$", when used in conjunction with a chemical moiety, such as, acyl, acyloxy, alkyl, alkenyl, alkynyl, or alkoxy is meant to include groups that contain from x to y carbons in the chain. $C_0$alkyl indicates a hydrogen where the group is in a terminal position, a bond if internal. A $C_{1-6}$alkyl group, for example, contains from one to six carbon atoms in the chain.

The term "alkylamino", as used herein, refers to an amino group substituted with at least one alkyl group.

The term "alkylthio", as used herein, refers to a thiol group substituted with an alkyl group and may be represented by the general formula alkylS-.

The term "amide", as used herein, refers to a group

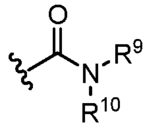,

wherein $R^9$ and $R^{10}$ each independently represent a hydrogen or hydrocarbyl group, or $R^9$ and $R^{10}$ taken together with the N atom to which they are attached complete a heterocycle having from 4 to 8 atoms in the ring structure.

The terms "amine" and "amino" are art-recognized and refer to both unsubstituted and substituted amines and salts thereof, e.g., a moiety that can be represented by

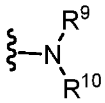 or 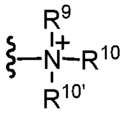,

wherein $R^9$, $R^{10}$, and $R^{10'}$ each independently represent a hydrogen or a hydrocarbyl group, or $R^9$ and $R^{10}$ taken together with the N atom to which they are attached complete a heterocycle having from 4 to 8 atoms in the ring structure.

The term "aminoalkyl", as used herein, refers to an alkyl group substituted with an amino group.

The term "aralkyl", as used herein, refers to an alkyl group substituted with an aryl group.

The term "aryl" as used herein include substituted or unsubstituted single-ring aromatic groups in which each atom of the ring is carbon. Preferably the ring is a 5- to 7-membered ring, more preferably a 6-membered ring. The term "aryl" also includes polycyclic ring systems having two or more cyclic rings in which two or more carbons are common to two adjoining rings wherein at least one of the rings is aromatic, e.g., the other cyclic rings can be cycloalkyls, cycloalkenyls, cycloalkynyls, aryls, heteroaryls, and/or heterocyclyls. Aryl groups include benzene, naphthalene, phenanthrene, phenol, aniline, and the like.

The term "carbamate" is art-recognized and refers to a group

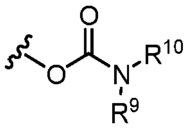 or 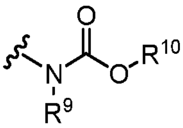,

wherein $R^9$ and $R^{10}$ independently represent hydrogen or a hydrocarbyl group.

The term "carbocyclylalkyl", as used herein, refers to an alkyl group substituted with a carbocycle group.

The term "carbocycle" includes 5-7 membered monocyclic and 8-12 membered bicyclic rings. Each ring of a bicyclic carbocycle may be selected from saturated, unsaturated and aromatic rings. Carbocycle includes bicyclic molecules in which one, two or three or more atoms are shared between the two rings. The term "fused carbocycle" refers to a bicyclic carbocycle in which each of the rings shares two adjacent atoms with the other ring. Each ring of a fused carbocycle may be selected from saturated, unsaturated and aromatic rings. In an exemplary embodiment, an aromatic ring, e.g., phenyl, may be fused to a saturated or unsaturated ring, e.g., cyclohexane, cyclopentane, or cyclohexene. Any combination of saturated, unsaturated and aromatic bicyclic rings, as valence permits, is included in the definition of carbocyclic. Exemplary "carbocycles" include cyclopentane, cyclohexane, bicyclo[2.2.1]heptane, 1,5-cyclooctadiene, 1,2,3,4-tetrahydronaphthalene, bicyclo[4.2.0]oct-3-ene, naphthalene and adamantane. Exemplary fused carbocycles include decalin, naphthalene, 1,2,3,4-tetrahydronaphthalene, bicyclo[4.2.0]octane, 4,5,6,7-tetrahydro-1H-indene and bicyclo[4.1.0]hept-3-ene. "Carbocycles" may be substituted at any one or more positions capable of bearing a hydrogen atom.

The term "carbocyclylalkyl", as used herein, refers to an alkyl group substituted with a carbocycle group.

The term "carbonate" is art-recognized and refers to a group $-OCO_2-$.

The term "carboxy", as used herein, refers to a group represented by the formula $-CO_2H$.

The term "ester", as used herein, refers to a group $-C(O)OR^9$ wherein $R^9$ represents a hydrocarbyl group.

The term "ether", as used herein, refers to a hydrocarbyl group linked through an oxygen to another hydrocarbyl group. Accordingly, an ether substituent of a hydrocarbyl group may be hydrocarbyl-O-. Ethers may be either symmetrical or unsymmetrical. Examples of ethers include, but are not limited to, heterocycle-O-heterocycle and aryl-O-heterocycle. Ethers include "alkoxyalkyl" groups, which may be represented by the general formula alkyl-O-alkyl.

The terms "halo" and "halogen" as used herein means halogen and includes chloro, fluoro, bromo, and iodo.

The terms "hetaralkyl" and "heteroaralkyl", as used herein, refers to an alkyl group substituted with a hetaryl group.

The terms "heteroaryl" and "hetaryl" include substituted or unsubstituted aromatic single ring structures, preferably 5- to 7-membered rings, more preferably 5- to 6-membered rings, whose ring structures include at least one heteroatom, preferably one to four heteroatoms, more preferably one or two heteroatoms. The terms "heteroaryl" and "hetaryl" also include polycyclic ring systems having two or more cyclic rings in which two or more carbons are common to two adjoining rings wherein at least one of the rings is heteroaromatic, e.g., the other cyclic rings can be cycloalkyls, cycloalkenyls, cycloalkynyls, aryls, heteroaryls, and/or heterocyclyls. Heteroaryl groups include, for example, pyrrole, furan, thiophene, imidazole, oxazole, thiazole, pyrazole, pyridine, pyrazine, pyridazine, and pyrimidine, and the like.

The term "heteroatom" as used herein means an atom of any element other than carbon or hydrogen. Preferred heteroatoms are nitrogen, oxygen, and sulfur.

The term "heterocyclylalkyl", as used herein, refers to an alkyl group substituted with a heterocycle group.

The terms "heterocyclyl", "heterocycle", and "heterocyclic" refer to substituted or unsubstituted non-aromatic ring structures, preferably 3- to 10-membered rings, more preferably 3- to 7-membered rings, whose ring structures include at least one heteroatom, preferably one to four heteroatoms, more preferably one or two heteroatoms. The terms "heterocyclyl" and "heterocyclic" also include polycyclic ring systems having two or more cyclic rings in which two or more carbons are common to two adjoining rings wherein at least one of the rings is heterocyclic, e.g., the other cyclic rings can be cycloalkyls, cycloalkenyls, cycloalkynyls, aryls, heteroaryls, and/or heterocyclyls. Heterocyclyl groups include, for example, piperidine, piperazine, pyrrolidine, morpholine, lactones, lactams, and the like.

The term "hydrocarbyl", as used herein, refers to a group that is bonded through a carbon atom that does not have a =O or =S substituent, and typically has at least one carbon-hydrogen bond and a primarily carbon backbone, but may optionally include heteroatoms. Thus, groups like methyl, ethoxyethyl, 2-pyridyl, and even trifluoromethyl are considered to be hydrocarbyl for the purposes of this application, but substituents such as acetyl (which has a =O substituent on the linking carbon) and ethoxy (which is linked through oxygen, not carbon) are not. Hydrocarbyl groups include, but are not limited to aryl, heteroaryl, carbocycle, heterocycle, alkyl, alkenyl, alkynyl, and combinations thereof.

The term "hydroxyalkyl", as used herein, refers to an alkyl group substituted with a hydroxy group.

The term "lower" when used in conjunction with a chemical moiety, such as, acyl, acyloxy, alkyl, alkenyl, alkynyl, or alkoxy is meant to include groups where there are ten or fewer atoms in the substituent, preferably six or fewer. A "lower alkyl", for example, refers to an alkyl group that contains ten or fewer carbon atoms, preferably six or fewer. In certain embodiments, acyl, acyloxy, alkyl, alkenyl, alkynyl, or alkoxy substituents defined herein are respectively lower acyl, lower acyloxy, lower alkyl, lower alkenyl, lower alkynyl, or lower alkoxy, whether they appear alone or in combination with other substituents, such as in the recitations hydroxyalkyl and aralkyl (in which case, for example, the atoms within the aryl group are not counted when counting the carbon atoms in the alkyl substituent).

The terms "polycyclyl", "polycycle", and "polycyclic" refer to two or more rings (e.g., cycloalkyls, cycloalkenyls, cycloalkynyls, aryls, heteroaryls, and/or heterocyclyls) in which two or more atoms are common to two adjoining rings, e.g., the rings are "fused rings". Each of the rings of the polycycle can be substituted or unsubstituted. **In** certain embodiments, each ring of the polycycle contains from 3 to 10 atoms in the ring, preferably from 5 to 7.

The term "sulfate" is art-recognized and refers to the group $-OSO_3H$, or a pharmaceutically acceptable salt thereof.

The term "sulfonamide" is art-recognized and refers to the group represented by the general formulae

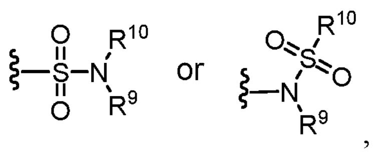

wherein $R^9$ and $R^{10}$ independently represents hydrogen or hydrocarbyl.

The term "sulfoxide" is art-recognized and refers to the group-S(O)-.

The term "sulfonate" is art-recognized and refers to the group $SO_3H$, or a pharmaceutically acceptable salt thereof.

The term "sulfone" is art-recognized and refers to the group $-S(O)_2-$.

The term "substituted" refers to moieties having substituents replacing a hydrogen on one or more carbons of the backbone. It will be understood that "substitution" or "substituted with" includes the implicit proviso that such substitution is in accordance with permitted valence of the substituted atom and the substituent, and that the substitution results in a stable compound or nanoparticle, e.g., which does not spontaneously undergo transformation such as by rearrangement, cyclization, elimination, etc. As used herein, the term "substituted" is contemplated to include all permissible substituents of organic compounds. In a broad aspect, the permissible substituents include acyclic and cyclic, branched and

unbranched, carbocyclic and heterocyclic, aromatic and non-aromatic substituents of organic compounds. The permissible substituents can be one or more and the same or different for appropriate organic compounds. For purposes of this invention, the heteroatoms such as nitrogen may have hydrogen substituents and/or any permissible substituents of organic compounds described herein which satisfy the valences of the heteroatoms. Substituents can include any substituents described herein, for example, a halogen, a hydroxyl, a carbonyl (such as a carboxyl, an alkoxycarbonyl, a formyl, or an acyl), a thiocarbonyl (such as a thioester, a thioacetate, or a thioformate), an alkoxyl, a phosphoryl, a phosphate, a phosphonate, a phosphinate, an amino, an amido, an amidine, an imine, a cyano, a nitro, an azido, a sulfhydryl, an alkylthio, a sulfate, a sulfonate, a sulfamoyl, a sulfonamido, a sulfonyl, a heterocyclyl, an aralkyl, or an aromatic or heteroaromatic moiety. It will be understood by those skilled in the art that the moieties substituted on the hydrocarbon chain can themselves be substituted, if appropriate.

The term "thioalkyl", as used herein, refers to an alkyl group substituted with a thiol group.

The term "thioester", as used herein, refers to a group $-C(O)SR^9$ or $-SC(O)R^9$
wherein $R^9$ represents a hydrocarbyl.

The term "thioether", as used herein, is equivalent to an ether, wherein the oxygen is replaced with a sulfur.

The term "urea" is art-recognized and may be represented by the general formula

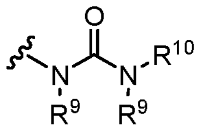
,

wherein $R^9$ and $R^{10}$ independently represent hydrogen or a hydrocarbyl.

The phrase "pharmaceutically acceptable" is art-recognized. **In** certain embodiments, the term includes compositions, excipients, adjuvants, polymers and other materials and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

"Pharmaceutically acceptable salt" or "salt" is used herein to refer to an acid addition salt or a basic addition salt which is suitable for or compatible with the treatment of patients.

The phrase "pharmaceutically acceptable carrier" as used herein means a pharmaceutically acceptable material, composition or vehicle, such as a liquid or solid filter, diluent, excipient, solvent or encapsulating material useful for formulating a drug for medicinal or therapeutic use.

As used herein, "weight average molecular weight" refers to the average molecular weight of a polymer having molecules of different chain lengths, as expressed by the equation:

$$M_w = \frac{\sum_i N_i M_i^2}{\sum_i N_i M_i}$$

where $N_i$ is the number of molecules of molecular weight $M_i$. The weight average molecular weight can be determined, for example, by light scattering, small angle neutron scattering, X-ray scattering, and sedimentation velocity.

As used herein, "number average molecular weight" refers to the average molecular weight of a polymer having molecules of different chain lengths, as expressed by the equation:

$$M_n = \frac{\sum_i N_i M_i}{\sum_i N_i}$$

where $N_i$ is the number of molecules of molecular weight $M_i$. The number average molecular weight can be determined, for example, by gel permeation chromatography (also known as size exclusion chromatography) or viscometry.

As used herein, the terms "polydispersity index" and "molecular distribution" are used interchangeably to refer to the ratio of the weight average molecular weight to the number average molecular weight.

As used herein, the term "distal end of the hydrophilic block" or "distal end of the hydrophobic block" are used to

refer to an end of a certain block that is not connected to the other block of the block copolymer, as illustrated by the example below:

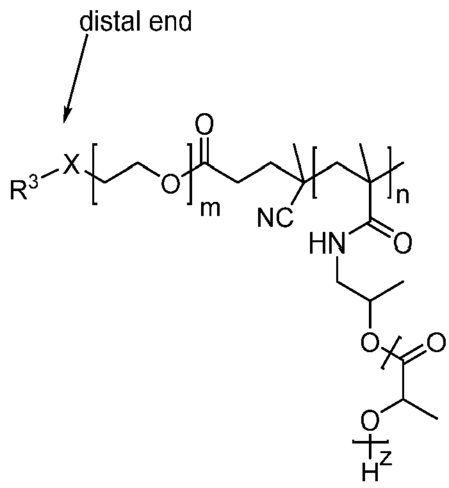

## EXAMPLES

The invention now being generally described, it will be more readily understood by reference to the following examples, which are included merely for purposes of illustration of certain aspects and embodiments of the present invention, and are not intended to limit the invention. Examples that do not disclose the subject-matter of the claims are to be considered as reference examples for illustrative purposes only.

**Experimental Design and Methods:**

PEG-b-p(HPMA$_m$-Lac$_n$) block copolymers may be synthesized by radical polymerization using HPMA$_m$-Lac$_n$ as monomer and PEG$_2$-ABCPA as macroinitiator by the following general protocol. HPMA$_m$-Lac$_n$ and PEG$_2$-ABCPA are dissolved at a total concentration of 0.3 g/mL in acetonitrile. To obtain block copolymers with different pHPMA$_m$-Lac$_n$ block lengths, the ratio of monomer to macroinitiator is varied between 35/1 to 150/1 (mol/mol). The polymerization is conducted at 70 °C for 24 h in a nitrogen atmosphere. Micellar formulation optimization is then carried out by using various concentrations of synthesized block copolymers PEG-b-p(HPMAm-co-Lac$_{4-8}$) from 10 to 30 mg/ml and drug concentration ranging from 0.25 to 2 mg/ml. Micellar formation is carried out by dropwise addition of organic drug-polymer solution to an aqueous phase composed of 150 mM ammonium acetate buffer (pH: 5). LE and CPX are loaded at the core of the micelles. The size and surface charge density of the micellar formulations are measured using Dynamic Light Scattering (DLS) and Zeta Potential analysis using a Malvern Zetasizer. The drug loading efficiency and amount of drug released at each time point are analyzed using High-Performance Liquid Chromatography (HPLC) technique. Validated HPLC methods are developed specifically for LE and CPX quantification. Based on these experiments, micellar formulations with sizes between 40-120 nm and polydispersity index (PDI) < 0.05 are selected for both groups. The drug loading capacity of optimal formulation should be above 5%. The predefined drug release duration for CPX is designed to be within 3 days and for LE to be 5-7 days full drug release profile.

To obtain surface modified (targeting) micelles, a protocol based on the synthesis of mPEG$_2$-ABCPA is developed. Briefly, di-tert-butyl decarbonate (t-Boc) protected macroinitiator (Boc-NH-PEG)$_2$-ABCPA) is de-protected following the method described previously. The free amine groups at the distal ends of macroinitiator (NH$_2$-PEG)$_2$-ABCPA is then reacted with 4-carboxyphenylbronic acid or SMCC. The rationale behind this route is that PBA or Mal conjugated PEG$_2$-ABCPA initiate free radical polymerization to form block copolymers. This allows a straightforward approach to obtain functionalized polymers that can be used for micellar formulation, without the need of post-modification procedures. To confirm the end group modification of synthesized block co-polymer with targeting molecules (i.e. PBA and/or Mal), $^1$HNMR spectroscopy and gel permeation chromatography (GPC) analysis are performed. For formulation of MTPs, various ratios of end group modified (targeting) polymers (i.e. PB/Mal-NH-PEG-b-p(HPMAm-co-Lac$_{4-8}$)) and non-modified (stealth) polymers PEG-b-p(HPMAm-co-Lac$_{4-8}$)) are used. The ratios of targeting: stealth polymers are 1:3, 2:3 and 1:1 in order to find the optimum density of targeting molecules on the surface of micelles to reach the highest cell or tissue binding. The micelles are then fluorescently labeled using fluorescein dye.

*In vitro* mucoadhesive (i.e. epithelial cell binding) properties of fluorescein labeled (non-drug loaded) targeting and non-targeting (control) micelles may be determined using an *in vitro* retention experiment by the following general

protocol. To do this, human corneal epithelial cells (HCEpC) are seeded on poly-lysine coated glass slides (25mm$^2$) at the density of 4×10$^4$ cells, followed by exposing cells to the eye drop solution composed of sequential concertation of micelles (from 5 to 20 mg/ml) at the rate of 1 mg mL$^{-1}$ for 1 h at 37 °C, followed washing steps with Dulbecco's phosphate-buffered saline (DPBS). Both targeting and non-targeting micelles are tested. For *ex vivo* experiments, same method is used but this time explanted corneal tissue is exposed to the particles solution **(FIG. 5).** The fluorescence intensity of micelles adhered to the mucin chains of HCEpC or corneal tissue is analyzed and quantified at different incubation time intervals and different micellar concentration at 37°C using a fluorescence microscopy and ImageJ software. Based on the collected data, the binding affinity (*Kd*) of the targeting molecules on the surface of the micelles is calculated. In addition, PrestoBlue and live/dead assays are used to assess the viability of HCEpC incubated with micellar formulations at various concentrations in order to check toxicity at different micellar concentrations.

The matrix solution (Non-Newtonian solution) with specified shear thinning and viscoelastic properties may be optimized by using different concentrations of Polycarbophil (0.1 to 2.0 %w/v) and EDTA (0.2 to 1 %w/v) in demineralized water containing 0.4 %w/v sodium chloride by the following general protocol. The concentrations of Tyloxapol (0.5 %w/v), Glycerin (0.5 %w/v) and benzalkonium chloride (0.003 %w/v) are kept constant. A rheometer (Anton Paar) is used to measure the rheological properties of the formulations using a cone plate (radius 8mm, cone angle 2°). A solvent trap is used to minimize water evaporation during measurement. Oscillatory rheology of formulations is applied at 25 °C using a 10 rad/s. Thixotropy (shear thinning) property of different formulations are assessed in a systematic way at 10 rad/s frequency and 1% strain (0.2 Pa oscillatory stress). A high strain (1000% = 30 Pa oscillatory stress) is applied for 1 min to convert the formulation to a sol. The raw data acquired by the rheometer is exported to an Excel file for plotting. Herschel-Bulkely regression model is applied for fitting the viscosity curves. The G-crossover point for different formulations indicate the transition from gel-state to sol-state. Selection criteria for optimum matrix solution is to achieve viscosity of 90-100 Pa at high shear stress (which is about 60× the viscosity of water). As the shear stress decreases, viscosity will increase, approaching infinity near the yield stress (about 2-4 Pa for optimum formulation). Below the yield stress, the optimum formulation will not flow (shear rate goes to zero).

After optimization of the matrix solution, the optimal formulation of micelles is dispersed in the matrix solution to form an eye drop for sustained release of drugs. The *in vitro* biocompatibility of the formulated eye drop using HCEpC is evaluated. In addition, DSL analysis is performed to evaluate size and PDI of micelles in the final eye drop formulation. *In vitro and ex vivo* retention experiments are performed.

Cornea scaffolds and bacterial cultures may be prepared by the following general protocol. Freshly harvested New Zealand white rabbit corneas (n=72) are excised and filled with 2%- agar solution to maintain cornea shape and cell viability, and media are added until the limbus. Gram positive *Staphylococcus aureus* (ATCC® 25923™) and gram-negative *Pseudomonas aeruginosa* (ATCC® 27853™) are utilized. Bacterial inoculation of corneas is performed with modification of a previously described protocol by wounding corneas with a scalpel to create a grid of 3 slashes vertically and horizontally, and 1.0 x 10$^4$ CFU (colony forming units) of either bacteria is inoculated directly to grid centers. Infected corneas are divided into a prophylactic and a treatment antibacterial efficacy study **(FIG. 7A).** The prophylactic (therapy immediately after inoculation) study consist of three groups per bacteria (n=6/group): i) no treatment, **ii)** 0.3% CPX 4x /day and **iii)** MTP antibiotic formulation 1x/ day. The treatment (therapy after 24 h of incubation) study consist of three groups per bacteria (n=6/group): **i)** no treatment, **ii)** 0.3% CPX 8x/day and iii) MTP antibiotic formulation 2x/ day. 24 h after initial drug administration, antibacterial efficacy is assessed through standard bacterial counts of the homogenized corneas. **In vivo:** The MTP matrix solution is tested *in vivo* using C57BL/6 mice for *P. aeruginosa* (ATCC® 19660™) and BALB/c mice for *S. aureus* (8325-4) in a prophylactic efficacy study. Left eyes are scarified with a 25-gauge needle with three 1 mm slashes penetrating only as deep as the superficial stroma and 1.0 x 10$^6$ CFU of *P. aeruginosa* or 1.0 x 10$^8$ of *S. Aureus* are applied on corneas. Corneas are assigned to the following groups (n=10/ group): **i)** no treatment, **ii)** 0.3% CPX 4x /day, and **iii)** MTP antibiotic formulation 1x/ day **(FIG. 7A).** Treatment is applied following bacterial inoculation. All assignments are performed randomly and include a 1:1 ratio of male and female mice. All animals are on the treatment regimen for 3 days.

*Data Collection and Analyses:* ***Ex vivo:*** Antibacterial efficacy is assessed with standard bacterial counts of corneal digests after 24 h. Bacterial counts are analyzed using t-test. ***In vivo:*** Mice are examined with the slit lamp biomicroscope at days 0, 1, 2, and 3 using a previously described method to assess keratitis graded from 0 to 4+, rating from clear or slightly opaque to perforation. To assess therapeutic efficacy, 6 samples are utilized for CFU counts of the corneal digests at day 1 and 3 to measure the antibacterial effects between the non-treated and therapeutic groups. Additionally, 4 samples from each group are utilized for H&E staining.

The optimized anti-inflammatory formulation may be evaluated in an *in vivo* murine inflammation model by the following general protocol. Inflammation is induced using the tip of a hand-held cautery by creating five burns to the central 50% of the right cornea of 48 deeply anesthetized C57BL/6 mice. The animals are assigned to two groups (n=30/group): i) early treatment in which mice start receiving treatment on day 0 and **ii)** late treatment in which the animals receive treatment on day 3, when the inflammatory cytokine levels peak. The early treatment group is divided into 3 subgroups (n=10/subgroup): **i)** no treatment, **ii)** 0.5% LE ophthalmic solution 4x/ day, and **iii)** MTP anti-inflammatory formulation 1x/ day. The late treatment group is divided into 3 subgroups (n=10/ subgroup): **i)** no treatment, **ii)** 0.5% LE ophthalmic solution

4x/ day, and **iii)** MTP anti-inflammatory formulation 2x/ day (**FIG. 8**). All assignments are performed randomly while maintaining an equal number of male and female mice within each group and its subgroups.

*Data Collection and Analyses:* To assess the anti-inflammatory efficacy of the treatments, corneas are excised for RT-PCR, IHC, and TUNEL assay. These methods evaluate cytokine levels associated with corneal inflammation (IL-1, IL-6, and TNF-$\alpha$), immune cell infiltrate (CD45+), and ocular surface cell apoptosis respectively. 6 samples are utilized for RT-PCR, and 4 samples for histological studies. Cytokine levels are analyzed using ANOVA and Tukey post-test.

**Example 1. Development, characterization, and assessment of biodegradable carrier systems with targeting sustained drug release properties.**

Amphiphilic block copolymers of polyethylene glycol-b-(N-(2-hydroxypropyl) methacrylamide-co-oligolactate) (PEG-b-p($HPMA_m$-co-$Lac_n$)) have been synthesized. The synthesized block copolymer is composed of a hydrophilic block of $PEG_{5000}$, which forms the micellar shell and a hydrophobic block composed of N-(2 hydroxypropyl)methacrylamide derivatized with various monodisperse oligolactide ($HPMA_m$-$Lac_n$), which acts as host for accommodating hydrophobic drug molecules (e.g. Loteprednol etabonate (LE) or Ciprofloxacin freebase (CPX)) **(FIG. 2).**

*Modifying the length of lactate side chains of mPEG-b-p(HPMAm-Lacn) block co-polymer for sustained drug release*

When the hydrophobic block of synthesized co-polymer is composed of only one or two lactate units p($HPMA_m$-$Lac_{1-2}$), the micelle formation is driven by thermosensitive block (i.e. p($HPMA_m Lac_{1-2}$)) dehydration and core formation (i.e. nucleation) above the cloud point temperature (CP) of the polymer. Below the CP, the amide group of p($HPMA_m Lac_{1-2}$) block has rotational freedom and may occur in both cis and trans conformation, whereas above the CP the mobility of the polymer chain is restricted due to stable trans conformation of amide group and possibly the intramolecular hydrogen bonding between the adjacent amide groups. However, when the number of lactate units increases to ~ 4-8 (i.e. $pHPMA_m Lac_{4-8}$), the micelle formation is mainly driven by hydrophobic interactions and self-assembly of lactate chains in the aqueous phase. In general, the drug molecules are released from the core of micelles via diffusion forces. Therefore, by changing the molar feed ratio of N-(2-hydroxypropyl) methacrylamide (HPMA) to lactide during the monomer ($HPMA_m$-$Lac_n$) synthesis step, the length of lactate chain can be fine-tuned, which will enable the formulation of micelles with tunable sustained release properties.

PEG-b-p(HPMAm-co-$Lac_{4-8}$) by radical polymerization has been synthesized using polyethylene $glycol_2$-4,4'-azobis(4 cyanopentanoic acid) ($PEG_2$-ABCPA) as macroinitiator and HPMAm-co-$Lac_{4-8}$ as monomer (ratio of monomer/initiator 150:1 mol/mol). LE was then loaded at the core of the micelles. Various ratios of polymer: drug (e.g. 10:0.1, 10:0.5, 10:1.0 and 10:2.0) were applied to optimize the formulation. The obtained preliminary results showed high stability of formulated micelles up to 7 days upon incubation in buffer solution at 37 °C. The physicochemical properties of LE loaded micelles are summarized in **FIG. 3A.** The *in vitro* release study showed a sustained release of LE (following first order release kinetics) during the total time period of 10 days **(FIG. 3B).** Regarding the drug loading capacity, there was a direct correlation between the amount of drug added and the final concentration of the loaded drug at the core of micelles **(FIG. 3C).**

The presence of a lactate polymer other than monolactate and dilactate (i.e. the presence of trilactate, tetra lactate or higher) may be useful for providing a polymer (blend) with a relatively low cloud point (CP), (in particular a CP below 20° C), thus providing a blend or copolymer (aggregate) with improved stability under ambient conditions. For such purposes, a tetralactate polymer is considered particularly suitable. Good results have been achieved with a copolymer, in particular a pHPMAm-dilactate, wherein at least 5% of the monomers are other than mono- and dilactate. For good water solubility not more than 22% of the monomers are other than mono- and dilactate. Further, a polymer with one or more lactate side chains of at least three lactic acid units (trilactate), in particular of at least four lactic acid units (tetralactate) has been found suitable to provide micelles of the polymer with improved stability, compared to the analogous polymer comprising only mono- or dilactate units. It should be noted that this in particular holds true for polymers further comprising a hydrophilic group (e.g. PEG).

*Surface modification of micelles with mucus targeting moieties and in vitro characterization*

The second component of this delivery platform is based on applying targeting moieties on the surface of micelles to render them site specific and to achieve immobilization via their covalent bonding on the surface of the corneal epithelial cells. The presence of amines within the mucin structure will lead to interaction of adjacent amino groups with the boronic acid groups to form an ionized borate which can significantly improve the complexation ability of phenylboronic acid with diols of glycoproteins (i.e. mucins) under physiological conditions (pH: 6-7). Apart from sialic group of adherent glycoproteins on the surface of corneal epithelial cells, there are plenty of cysteine-rich subdomains within the glycoproteins structure. As an alternative targeting moieties, maleimide (Mal) exhibits high selectivity and reactivity to cysteine

molecule appeared on protein structure through a Michael type addition reaction. Other advantage with Mal group is unlike thiomers, they are not predisposed to oxidative dimerization due to the formation of disulfide bonds. For this reason, two types of epithelial mucin targeting moieties (PBA and Mal) will be studied and compared for their binding affinity to corneal epithelial mucin (FIG. 4A).

The reactive amine groups at the distal ends of the PEG chains (($NH_2$-PEG)$_2$-ABCPA) were protected with di-tert-butyl decarbonate (t-Boc) to form ((Boc-NH-PEG)$_2$-ABCPA) **(FIG. 4B).** The integration of $^1$HNMR peaks confirmed the presence of two Boc molecules at the two distal ends of PEG chains. Next, the Boc groups are deprotected to provide active amine groups accessible for conjugation with Succinimidyl-4-(N-maleimidomethyl) cyclohexane-1-carboxylate (SMCC) or 4-carboxyphenylbronic acid.

**Example 2. Formulation and optimization of micelles' matrix solution as an eyedrop product**

Based on the designed system, the drug loaded micelles **1)** homogenously deliver to the ocular surface upon each instillation, and **2)** penetrate efficiently through the mucus layer and interact with the adherent mucin molecules on the surface of corneal epithelial cells via stable covalent bond formation. Therefore, a matrix solution, in which micelles will be embedded, further enhances the residence time of the micellar formulation (passive retention of micelles) at the ocular surface upon instillation. An increase in contact time will increase the retention of micelles (via covalent bond formation) on the surface of tissue. The matrix solution will be composed of Polycarbophil, Tyloxapol, and Edetate disodium dihydrate (EDTA). The formulation will have specific viscoelastic properties (non-settling gel) in bottle due to mutual charge repulsion of Polycarbophil chains. Once applied as eyedrop, the electrolytes in the tear fluid decrease the swelling of the polymer by partial screening of the charged repulsion, converting the gel into a viscous liquid which can spread homogenously over the eye via blinking. The main roles of Polycarbophil are that upon instillation **1)** the polymer chains can undergo interpenetration with the glycoprotein chains of the mucus on the cornea surface, **2)** it forms hydrogen-bonding with the corneal and conjunctival mucus, both phenomena will help in extending the passive retention of micelles to several hours. Tyloxapol helps in clearing out the floating nonadherent mucin layer while the EDTA acts as chelating agent, playing main role in maintaining the viscosity of matrix solution before and after instillation on the corneal tissue. Most importantly, it helps in washing away the deposited calcium ions on the surface of the mucus layer which accelerates the penetration of micelles towards the epithelium.

Matrix solutions based on varying concentrations of polycarbophil from 0.1 to 2.0 %w/v were formulated with fixed concentrations of Tyloxapol (0.5 %w/v), and EDTA (1 %w/v) in demineralized water containing 0.4 %w/v sodium chloride supplemented with glycerin (0.5 %w/v) and benzalkonium chloride (0.003 %w/v). The pH of the final solution was adjusted to 6.5 using 0.1 M sodium hydroxide. The viscosity for different formulations as a function of shear stress were assessed from 0.01 to 100 $s^{-1}$ at 25 °C. The obtained results showed that formulations containing more than 0.3 %w/v polycarbophil concentration have higher viscosity at low shear. The yield stress for the formulation containing 1.5 %w/v polycarbophil was about 15 Pa **(FIG. 6).** Below the yield stress, the gel did not flow, and hence, viscosity could not be measured. At high shear, the formulation had sufficiently low viscosity (>100 cps) that could be delivered as a drop.

**Example 3. To test the efficacy of mucus targeting eyedrop *ex vivo* and *in vivo.***

MTPs loaded with either the anti-inflammatory LE or the antibiotic CPX dispersed in the matrix solution (at the final polymer and drug concentrations of 30 mg/ml, 0.3%, and 0.5%, respectively) are prepared. The antibiotic formulation are assessed in an *ex vivo* rabbit eyeball model to validate the bactericidal efficacy, followed by a non-inferiority study of the formulation in an *in vivo* microbial model. The anti-inflammatory formulation are evaluated in a non-inferiority study on *in vivo* inflammation model.

*Establishing non-inferiority of the antibiotic loaded MTPs formulation compared to commercial product in ex vivo and in vivo models.*

Serious ocular injuries, such as those encountered by servicemen and women, pose a significant risk of infection to the inured eye. Both the infectious process, and the host immuno-inflammatory response can lead to tissue necrosis, leading to delayed wound healing and potentially permanent damage to vision. To prevent and treat such infections, broad-spectrum antibiotics such as fluoroquinolones have been shown to be effective against both gram-positive and gram-negative bacteria. Current standard of care requires a high dosage and high frequency of antibiotic eyedrops, such as 0.3% CPX ophthalmic solution with dosing as high as every 2 h. The therapeutic efficacy of the formulation disclosed in Examples 1 and 2 is assessed and compared to commercialized product *ex vivo* and *in vivo.*

*Establishing non-inferiority of the anti-inflammatory loaded MTPs formulation compared to commercialized product in vivo*

Serious ocular injuries are often followed by a fulminant local inflammatory response, which is often unregulated and can lead to significant tissue damage, delayed wound healing, cell loss, fibrosis/scarring, or even tissue necrosis and ultimately loss of vision. Even if no scarring develops, it is not uncommon for corneal injuries to lead to irregular astigmatism as a result of the loss of the normal contour of the eye. To suppress the inflammation, topical corticosteroids such as 0.5% LE are administered with complex regimen. Most commercially available formulations require high frequency dosing, 4 times a day, to achieve therapeutic levels. The therapeutic efficacy of the anti-inflammatory formulation disclosed in Examples 1 and 2 is assessed and compared to commercial product in an *in vivo* model.

The synthesis of (PBA/Mal-$PEG_2$-ABCPA) was performed in 3 major steps (Scheme 1).

**Example 4. Chemical Synthesis**

Boc protection of $NH_2$-PEG-OH

NH2 -PEG-OH (5000 or 3400Da) (1 g, 0.20 mmol) was dissolved in 10 mL DCM. Next, di-tert-butyl dicarbonate (t-Boc) (87.3 mg, 0.40 mmol) was added to the solution. The reaction mixture was stirred for 24 h under an $N_2$ atmosphere. Next, DCM was partially evaporated under reduced pressure and the obtained product (Boc-NHPEG-OH) was precipitated in 50 mL diethyl ether. The product was dissolved in DCM and precipitated diethyl ether (3 times).

Synthesis of $(Boc-NH-PEG)_2$ -ABCPA or $PEG_2$ -ABCPA

PEG-ABCPA- PEG or Boc-PEG-ABCPA-PEG-Boc macro-initiators were synthesized through an esterification of mPEG (molecular weight, 2.0 or 5.0 kDa) and ABCPA, using DCC as a coupling reagent and 4-(dimethylamino) pyridinium 4-toluenesulfonate (DPTS, which was made by separately dissolving DMAP and p-toluenesulfonic acid in tetrahydrofuran (THF) and mixing the two solutions using a 1:1 molar equivalence) as a catalyst (Scheme 1). ABCPA (1 equiv), mPEG (2 equiv), and DPTS (0.3 equiv) (or 0.280 g of ABCPA, 10 g of mPEG, 0.094 g of DPTS) were dissolved in 50 mL of dry dichloromethane (DCM) and put on ice. Next, 3 equiv of DCC (0.619 g of DCC) were dissolved in 50 mL of DCM and dropwise added to the mPEG solution under nitrogen atmosphere. After addition of DCC, the ice bath was removed allowing the reaction mixture to reach room temperature. After 16 h at room temperature, the reaction mixture was filtered to remove the precipitated 1,3-dicyclohexyl urea and the solvent was removed in vacuo. The product was dissolved in water, stirred for 2 h, and dialyzed against water for 72 h at 4 °C. The sample was freeze-dried to obtain a fluffy white product.

Deprotection of (Boc-NH-PEG)2 -ABCPA

(Boc-NH-PEG)2 -ABCPA (200 mg, 19.1 µmol) was deprotected in 2 mL DCM/ TFA 1/1 (v/v) mixture for 4 h at room temperature. Next, the solvent was partially evaporated under reduced pressure and the product (TFA·NH2 -PEG)2 -ABCPA was precipitated in diethyl ether, followed by dissolution in DCM and precipitated in diethyl ether (3 times).

Synthesis of (PBA-PEG)2 -ABCPA

A mixture of the 4-carboxyphenylbronic acid (0.81 mmol), the (TFA-NH2 -PEG)2 - ABCPA (1.0 mmol), and TEA (1.6 mmol) in DMF (4 mL) was treated with PyBOP (380 mg, 1.0 mmol). After 2 h, the reaction mixture was concentrated in vacuo, suspended in H2O (25 mL), and stirred at RT. After 16 h, the mixture was filtered, and the filter cake was washed with ether (2 x 2 mL).

Synthesis of (Mal-PEG)2 -ABCPA

A stock solution of Succinimidyl-4-(N-maleimidomethyl) cyclohexane-1-carboxylate (SMCC) (0.156 g, 0.5 mol) was prepared in 1 ml of pre-dried DCM, 6 eq of this stock solution was pipetted into a glass vial and 10 eq triethylamine in DCM was subsequently added. Next, a solution of PLGA-PEG-NH2.TFA (100 mg, 8.5 µmol, 1 eq) in 2 ml DCM was slowly added to this reaction mixture while stirring at room temperature. The reaction was allowed to proceed for 48 hours under an N2 atmosphere in the dark. The final product was precipitated in diethyl ether, followed by dissolution in DCM and reprecipitation in diethyl ether (3 times).

Synthesis of HPMA-$LAC_n$

A mixture of 1 equiv of L-lactide (1g), 1 equiv of HPMAm (0.993g), 0.01 equiv of SnOct 2 (28.1mg, 1mol% relative to HPMAm) were added in a round bottom flask. Vacuum and nitrogen alternating cycles were repeated three times to remove air. Then, the mixture was heated to 110 °C with stirring for 1 h and allowed to cool to room temperature. The purification was done through silica column chromatography. The reaction mixture was first dissolved in small amount of ethyl acetate (EtOAc) and dry-loaded on Silica column. 90% EtOAc/Hexane solvent system was used to run the column entirely. Thin Layer Chromotography (TLC) was used to analyze the separation. Fractions containing $HPMA_m$-$Lac_n$ were collected, and after solvent evaporation, the identity of obtained fractions was established by $^1$H-NMR in $CDCl_3$.

Synthesis of Copolymer PBA/Mal-PEG 3.4k -b-p($HPMA_m$-$Lac_n$)

PEG-b-poly($HPMA_m$-$Lac_n$) or PBA/Mal-PEG-b-poly($HPMA_m$-$Lac_n$) was synthesized by radical polymerization using PEG 2 -ABCPA as macroinitiator and HPMAm-Lac n as monomer. Monomer to macroinitiator feed ratio was 150:1. A mixture of HPMAm-Lacn and PEG 2-ABCPA were dissolved in dry acetonitrile (ACN). The concentration of macroinitiator plus monomer is 300mg/mL in ACN. The resulting solution was flushed with nitrogen for at least 15 mins and then heated to 70 °C with stirring for 24 hours. After 24 hours, the reaction was cooled to room temperature and diluted in small amount of ACN (~2 ml). The product in the solution was precipitated by dropwise addition to an excess of cold diethyl ether (~45 ml) in a 50 ml vial. After centrifugation at 3000 rpm for 15 minutes, white pellet was obtained. Diethyl ether wash followed by centrifugation was repeated three times. Followed by dissolution in water, and dialysis (membrane with cutoff 12-14kDa) against water and finally recovered by freeze drying. The final product was analyzed by GPC and $^1$H-NMR.

**Example 5. Polymer characterization**

Gel permeation chromatography (GPC) characterization of the polymers

Analysis of the $PEG_2$-ABCPA macroinitiator and PEG-b-poly($HPMA_m$-$Lac_n$) block copolymer was performed using a Waters System (Waters Associates Inc., Milford, MA) with refractive index (RI) using two serial Plgel 5 μm MIXED-D columns (Polymer Laboratories) and THF as eluent. The flow rate was 0.7 mL/min (45 min run time) and the temperature was 25 °C. The molecular weights of the synthesized polymers were determined by GPC analysis using RI detector and standards to calculate the number and weight average molecular weight (Mn, Mw) and polydispersity index (PDI; (Mw/Mn ).

$^1$H NMR characterization of the polymers

$^1$H NMR spectra in solution were recorded at 400 MHz unless otherwise noted. Chemical shifts are reported in ppm and the (residual) solvent signals of CDCl3 ($^1$H NMR: δ 7.26 ppm) were used as reference. The copolymer composition and its Mn (kDa) were determined as previously described.

**Example 6. Formulation of Loteprednol Etabonate (LE) loaded micelles**

Micelles were formed via combination of a 'solvent evaporation' procedure and a 'rapid heating' procedure. For the preparation of LE loaded micelles, the mPEG-b-p(HPMAm-$Lac_{2-4}$) block copolymers were dissolved in 1ml Acetone and the drug molecule (LE) at desired concentration was added from DMSO stock solution to the polymeric solution. After vortexing vigorously and incubation at 40 °C for 30 min, the organic solution was added fast to the aqueous solution at 1ml volume composed of ammonium acetate buffer pH 5 (120 mM) under stirring for 30 min at room temperature, following by an increase in temperature and stirring for another 2 hr at 45 °C. Subsequently, the solution was be stirred overnight at room temperature for evaporation of organic solvent. The nonentrapped, precipitated LE was removed by centrifugation (4000 rpm, 10 min).

**Example 7. Determination of loading efficiency and loading capacity**

The amount of loaded LE in the polymeric micelles was determined by diluting the different micellar dispersions (20ul) in Acetonitrile (180ul). Subsequently vortexing was done to destabilize the micelles and dissolve LE. The absorbance of the obtained solutions was measured at 243 nm using an HPLC method. CM dissolved in Acetonitrile was used for calibration (concentration from 0.0 mg/mL to 0.5 mg/mL). The encapsulation efficiency (EE) and loading capacity (LC) were calculated as follows:

$$EE = (\text{amount of measured LE/amount of LE added}) \times 100\%$$

LC = (amount of measured LE)/amount of (measured LE + polymer added) × 100%.

**Dynamic Light Scattering (DLS)**

Freshly prepared micellar dispersions were diluted 25 times with 10 mM HEPES, pH 7.0 (final concentration 400 μg/ml). The sizes of micelles were analyzed with a Malvern Zetasizer Nano dynamic light scattering. Standard operating procedure parameters: 10 runs, 10 seconds/run, three measurements, no delay between measurements, 25 °C with 120 second equilibration time. Collection parameters: S26 Lower limit = 0.6, Upper limit = 1000, Resolution = High, Number of size classes = 70, Lower size limit = 0.4, Upper size limit = 1000, Lower threshold = 0.05, Upper threshold = 0.01. Data is representative of three replicate measurements.

**Zeta potential**

Zeta potential of the micelles was determined using a Malvern Zetasizer Nano-Z (Malvern Instruments, Malvern, UK) with universal ZEN 1002 'dip' cells and DTS (Nano) software (version 4.20) at 25 °C. Zeta-potential measurements were performed in 10 mM HEPES at pH 7.4 at a final polymer concentration of 400 μg/mL.

**Example 8. *In vitro* release of LE from nanoparticles**

The release of LE from the polymeric micelles was examined by a dialysis method. In detail, 1 mL of LE loaded polymeric micelles in ammonium acetate buffer was pipetted into a pre-swollen dialysis bag (MWCO 15,000). A solution of 2% Triton X-100 in the same PBS buffer was used as releasing medium. The dialysis bag was immersed into 10 mL of the releasing medium with stirring at 500 rpm at 37 °C. Samples (5 mL) of the receiving medium were drawn periodically, and the volume was adjusted with fresh release medium. The concentration of LE in the different samples was measured at 244 nm using a HPLC method. Release samples were treated with Acetonitrile by making 2x dilutions and the calibration was obtained using LE (concentration from 0.0 mg/mL to 0.5 mg/mL) in Acetonitrile. At the end of the experiment, the amount of the remaining LE in polymeric micelles in dialysis bags was measured using HPLC method.

To obtain PBA or Mal targeted block copolymers, a protocol to synthesize bi-functionalized azo macronitiator ((PBA/Mal-PEG)2 -ABCPA) was developed, based on the synthesis of mPEG2 -ABCPA previously described by Neradovic et al[15]. The rationale behind this route is that the (PBA/Mal -PEG)2 -ABCPA will initiate free radical polymerization to form block copolymers with a PBA or Mal functionality at the distal PEG end **(FIG. 13).** Consequently, the PBA or Mal can be coupled to the macroinitiator, to allow a straightforward approach to obtain functionalized polymers that can be used for micellar preparation, without the need of post-modification procedures. The synthesis of (PBA/Mal-PEG)2 -ABCPA **(FIG. 9)** starts with Boc protection of the primary amine (a) of the commercially available NH2 -PEG-OH. Next, Boc-NH-PEG-OH was coupled to ABCPA via DCC coupling (b). GPC analysis of the product **(FIG. 10)** showed a bimodal distribution, corresponding to a mixture of PEG bi-functionalized ABCPA (7 kDa) and unreacted Boc-NH-PEG-OH or PEG mono-functionalized ABCPA (3.4 kDa). The ratio between 7 kDa and 3.4 kDa products was 77%/23% **(FIG. 11).** The third step of the reaction scheme was the Boc deprotection with TFA, generating the TFA salt form of (NH2 -PEG)2 -ABCPA (c). The last step of the synthesis was the reaction between NHS activated Mal (Mal-NHS) or 4-carboxyphenylbronic acid and the deprotected (TFA-NH2 PEG)2 ABCPA (d). The reaction was performed under anhydrous conditions to avoid hydrolysis and using a molar excess of Mal-NHS or 4-carboxyphenylbronic acid to NH2 groups from the macroinitiator to limit possible aminolysis of the ester bond that links the PEG chain and ABCPA.

The $^1$H NMR results showed the presence of 2 BOC groups at two ends of the PEG2-APCPA polymer.

$^1$H NMR and GPC results demonstrated that the applied synthetic route lead to the formation of first step (Boc-PEG)2 -ABCPA **(FIG. 12)**

Free radical polymerization of HPMA-$Lac_{1-2}$ and/or HPMA-$Lac_{2-4}$ with (PEG)2-ABCPA as macroinitiator (without targeting groups attached) resulted in the formation of PEG-b-p(HPMAm-$Lac_{2-4}$)) **(FIG. 14).** The yield of polymerizations was close to 70% (see **Table 1) (FIG. 16).**

**Table 1.** Overview of the characteristics of the synthesized stealth PEG-b-p(HPMAm-$Lac_{2-4}$) was determined by GPC and $^1H$ NMR.

| Polymer | GPC | | | $^1H$ NMR | | | Yield (%) |
|---|---|---|---|---|---|---|---|
| | $M_n$(kDa) | PDI | dn/dc | $M_n$(kDa) | Feed | Polymer | |
| PEG-b-p(HPMAm-$Lac_{2-4}$) | 29.68 | 1.4 | 0.10 | 38.44 | 150:1 | 96:1 | 64-70 |

## REFERENCES CITED

1. Ghate D, E.H., Ocular drug delivery. Expert Opinion on Drug Delivery, 2006. 3: p. 275-87.
2. Sears ML, e., Pharmacology of the Eye. Berlin Heidelberg: Springer-Verlag, 1984.
3. Bucolo, C., F. Drago, and S. Salomone, Ocular drug delivery: a clue from nanotechnology. Front Pharmacol, 2012. 3: p. 188.
4. V.H.L. Lee, J.R.R., Review: topical ocular drug delivery: recent developments and future challenges. J. Ocul. Pharmacol., 1986. 2: p. 67-108.
5. A. Topalkara, C.G.I., D.S. Arici, M.K. Arici, Adverse effects of topical antiglaucoma drugs on the ocular surface. Clin. Exp. Ophthalmol, 2000. 28: p. 113-117.
6. Nagarwal RC, K.S., Singh PN, Maiti P, Pandit JK, Polymeric nanoparticulate system: a potential approach for ocular drug delivery. Journal of Controlled Release, 2009. 136(1): p. 2-13.
7. Martin J Coffey, H.H.D., and Stephen S Lane, Development of a non-settling gel formulation of 0.5% loteprednol etabonate for anti-inflammatory use as an ophthalmic drop. Clin Ophthalmol., 2013. 7: p. 299-312.
8. Lai SK, W.Y., Hanes J., Mucus-penetrating nanoparticles for drug and gene delivery to mucosal tissues. Adv Drug Deliv Rev, 2009. 61(2): p. 158-71.
9. Hu Q, R.C., van Gaal E, Brundel P, Kostkova H, Etrych T, Weber B, Barz M, Kiessling F, Prakash J, Storm G, Hennink WE, Lammers T., Tailoring the physicochemical properties of corecrosslinked polymeric micelles for pharmaceutical applications. J Control Release, 2016. 244: p. 314-325.
10. Rijcken, C.J.F., Tunable and degradable polymeric micelles for drug delivery: from synthesis to feasibility invivo. 2007.
11. Fujii, N.K.a.S., Recent Advances in Ocular Drug Delivery Systems. Polymers, 2011. 3: p. 193-221.


## Claims

**1.** A pharmaceutical formulation, comprising:

a nanoparticle comprising an amphiphilic block copolymer; and
an aqueous matrix comprising an anionic polymer;
wherein the nanoparticle is dispersed in the aqueous matrix;
wherein the amphiphilic block copolymer has the following structure:

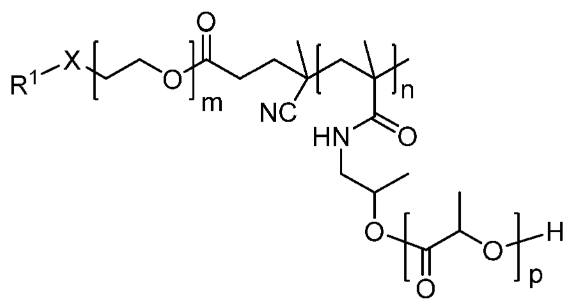

,

wherein X is O, NH, or S; $R^1$ is hydrogen, alkyl, alkenyl, alkynyl, carbocyclyl, heterocyclyl, aryl, or heteroaryl; m and n are each independently an integer from 5-2000; and p is an integer from 1 to 10.

**2.** The pharmaceutical formulation of claim 1, wherein the nanoparticle is a micelle.

3. The pharmaceutical formulation of claim 1, wherein the hydrophilic block has a number-average molecular weight of about 500 Da to about 20 kDa, or the hydrophilic block has a number-average molecular weight of about 1 kDa to about 8 kD.

4. The pharmaceutical formulation of claim 1,
wherein the hydrophobic block has a number average molecular weight of about 500 Da to about 200 kDa, optionally wherein the hydrophobic block has a number-average molecular weight of about 1 kDa to about 50 kDa, or a number-average molecular weight of about 2 kDa to about 20 kDa.

5. The pharmaceutical formulation of any one of claims 1-4, wherein the nanoparticle has a diameter of about 10 nm to about 1000 nm, preferably about 30 nm to about 500 nm, more preferably about 50 nm to about 250 nm, wherein the nanoparticle diameter is measured by a dynamic light scattering.

6. The pharmaceutical formulation of any one of claims 1-5, wherein the surface of the nanoparticle comprises a glycoprotein targeting moiety, optionally wherein the glycoprotein targeting moiety comprises a boronic acid group:

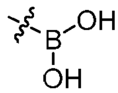,

or
wherein the glycoprotein targeting moiety comprises a maleimide group:

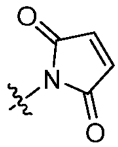,

preferably wherein the glycoprotein targeting moiety is coupled to the distal end (e.g., the amine, hydroxyl or thiol) of the hydrophilic block, preferably has the following structure:

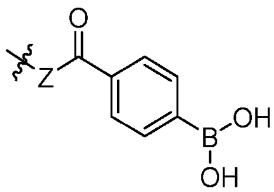,

or
the glycoprotein targeting moiety has the following structure:

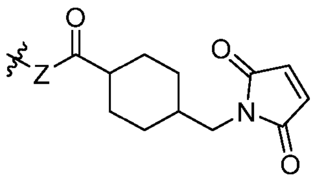,

wherein Z is O, NH, or S.

7. The pharmaceutical formulation of any one of claims 1-6, wherein the nanoparticle further comprises a therapeutic agent, optionally wherein the therapeutic agent is an antibacterial agent or an anti-inflammatory agent, preferably the antibacterial agent or anti-inflammatory agent is selected from loteprednol etabonate, dexamethasone, and ciprofloxacin.

8. The pharmaceutical formulation of any one of claims 1-7, wherein the anionic polymer is cross-linked, preferably wherein the cross-linked anionic polymer has a cross-linking density of from about 0.01% to about 2.0%, optionally wherein the cross-linking density of the anionic polymer is from about 0.05% to about 0.5%, preferably the anionic polymer comprises a repeat unit selected from

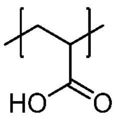 and 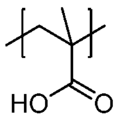,

or

the anionic polymer comprises a cross-linking unit derived from divinyl glycol, preferably wherein the anionic polymer is polycarbophil, more preferably the polycarbophil is calcium polycarbophil, or the matrix has a polycarbophil weight to volume percent *(%w/v)* of about 0.01% to about 10.0%, preferably a weight to volume percent *(%w/v)* of about 0.1% to about 2.0%.

9. The pharmaceutical formulation of any one of claims 1-8, wherein the aqueous matrix comprising the cross-linked ionic polymer is a non-settling gel, optionally wherein the aqueous matrix further comprises Tyloxapol, or the aqueous matrix has a Tyloxapol weight to volume percent *(%w/v)* of about 0.01% to about 5.0%, preferably weight to volume percent *(%w/v)* of about 0.1% to about 2.0%, more preferably weight to volume percent *(%w/v)* of about 0.5%, optionally wherein the aqueous matrix further comprises a chelating agent, preferably edetate disodium dihydrate (EDTA).

10. The pharmaceutical formulation of any one of claims 1-9, wherein the pharmaceutical formulation is formulated for topical administration, optionally wherein the pharmaceutical formulation is formulated for topical administration as an ophthalmic drop.

## Patentansprüche

1. Pharmazeutische Formulierung, umfassend:

   ein Nanopartikel, das ein amphiphiles Blockcopolymer umfasst; und
   eine wässrige Matrix, die ein anionisches Polymer umfasst;
   wobei das Nanopartikel in der wässrigen Matrix dispergiert ist;
   wobei das amphiphile Blockcopolymer die folgende Struktur aufweist:

   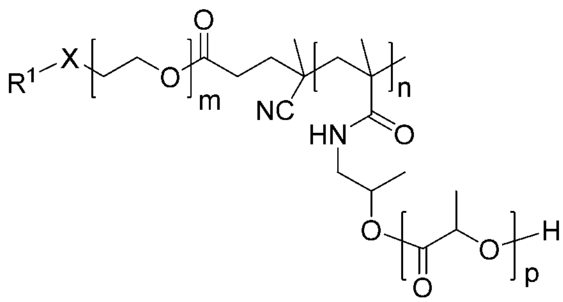,

   wobei X O, **NH** oder S ist; $R^1$ Wasserstoff, Alkyl, Alkenyl, Alkinyl, Carbocyclyl, Heterocyclyl, Aryl oder Heteroaryl ist; m und n jeweils unabhängig voneinander eine ganze Zahl von 5 bis 2000 sind; und p eine ganze Zahl von 1-10 ist.

2. Pharmazeutische Formulierung nach Anspruch 1, wobei das Nanopartikel eine Mizelle ist.

3. Pharmazeutische Formulierung nach Anspruch 1, wobei der hydrophile Block ein zahlengemitteltes Molekulargewicht von etwa 500 Da bis etwa 20 kDa aufweist oder der hydrophile Block ein zahlengemitteltes Molekulargewicht von etwa 1 kDa bis etwa 8 kD aufweist.

4. Pharmazeutische Formulierung nach Anspruch 1,
wobei der hydrophobe Block ein zahlengemitteltes Molekulargewicht von etwa 500 Da bis etwa 200 kDa aufweist, wobei der hydrophobe Block gegebenenfalls ein zahlengemitteltes Molekulargewicht von etwa 1 kDa bis etwa 50 kDa oder ein zahlengemitteltes Molekulargewicht von etwa 2 kDa bis etwa 20 kDa aufweist.

5. Pharmazeutische Formulierung nach einem der Ansprüche 1-4, wobei das Nanopartikel einen Durchmesser von etwa 10 nm bis etwa 1000 nm, vorzugsweise etwa 30 nm bis etwa 500 nm, noch bevorzugter etwa 50 nm bis etwa 250 nm aufweist, wobei der Nanopartikeldurchmesser durch dynamische Lichtstreuung gemessen wird.

6. Pharmazeutische Formulierung nach einem der Ansprüche 1-5, wobei die Oberfläche des Nanopartikels eine auf Glykoproteine gerichtete Einheit umfasst, wobei die auf Glykoproteine gerichtete Einheit gegebenenfalls eine Boronsäuregruppe umfasst:

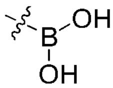

oder
wobei die auf Glykoproteine gerichtete Einheit eine Maleimid-Gruppe umfasst:

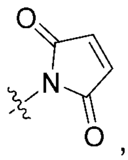 ,

vorzugsweise wobei die auf Glykoproteine gerichtete Einheit an das distale Ende (z.B. das Amin, Hydroxyl oder Thiol) des hydrophilen Blocks gekoppelt ist, vorzugsweise die folgende Struktur aufweist:

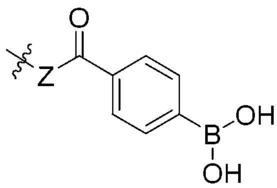

oder
die auf Glykoproteine gerichtete Einheit die folgende Struktur aufweist:

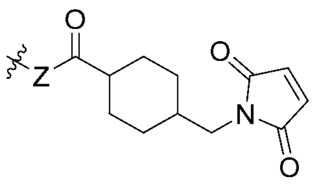 ,

wobei Z O, NH oder S ist.

7. Pharmazeutische Formulierung nach einem der Ansprüche 1-6, wobei das Nanopartikel ferner einen therapeutischen Wirkstoff umfasst, wobei der therapeutische Wirkstoff gegebenenfalls ein antibakterieller Wirkstoff oder ein entzündungshemmender Wirkstoff ist, wobei der antibakterielle Wirkstoff oder der entzündungshemmende Wirkstoff vorzugsweise aus Loteprednol-Etabonat, Dexamethason und Ciprofloxacin ausgewählt ist.

8. Pharmazeutische Formulierung nach einem der Ansprüche 1-7, wobei das anionische Polymer quervernetzt ist, vorzugsweise wobei das quervernetzte anionische Polymer eine Quervernetzungsdichte von etwa 0,01 % bis etwa 2,0 % aufweist, gegebenenfalls wobei die Quervernetzungsdichte des anionischen Polymers etwa 0,05 % bis etwa 0,5 % beträgt, wobei vorzugsweise das anionische Polymer eine sich wiederholende Einheit umfasst, die ausgewählt

ist aus

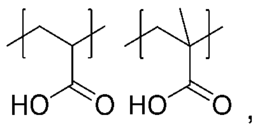

oder
das anionische Polymer eine Quervernetzungseinheit umfasst, die von Divinylglykol abgeleitet ist, wobei das anionische Polymer vorzugsweise Polycarbophil ist, noch bevorzugter das Polycarbophil Calciumpolycarbophil ist, oder die Matrix ein Polycarbophil-Gewichts-/Volumenprozent (%w/v) von etwa 0,01 % bis etwa 10,0 %, vorzugsweise ein Gewichts-/Volumenprozent (%w/v) von etwa 0,1 % bis etwa 2,0 % aufweist.

**9.** Pharmazeutische Formulierung nach einem der Ansprüche 1-8, wobei die wässrige Matrix, die das quervernetzte ionische Polymer umfasst, ein nicht sedimentierendes Gel ist, wobei die wässrige Matrix gegebenenfalls zusätzlich Tyloxapol umfasst oder die wässrige Matrix ein Tyloxapol-Gewichtsvolumenprozent (%w/v) von etwa 0,01 % bis etwa 5,0 %, vorzugsweise ein Gewichtsvolumenprozent (%w/v) von etwa 0,1 % bis etwa 2,0 %, vorzugsweise von etwa 0,5 % aufweist, wobei die wässrige Matrix gegebenenfalls zusätzlich einen Chelatbildner, vorzugsweise Edetat-Dinatrium-Dihydrat (EDTA), umfasst.

**10.** Pharmazeutische Formulierung nach einem der Ansprüche 1-9, wobei die pharmazeutische Formulierung zur topischen Verabreichung formuliert ist, wobei die pharmazeutische Formulierung gegebenenfalls zur topischen Verabreichung als ophthalmische Tropfen formuliert ist.

## Revendications

**1.** Formulation pharmaceutique comprenant :

une nanoparticule comprenant un copolymère à blocs amphiphile ; et
une matrice aqueuse comprenant un polymère anionique ;
dans laquelle la nanoparticule est dispersée dans la matrice aqueuse ;
dans laquelle le copolymère à blocs amphiphile a la structure suivante :

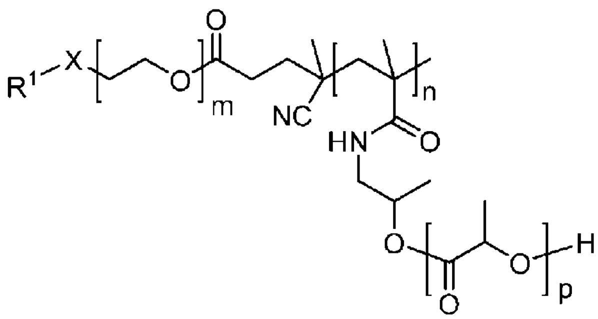

dans laquelle X est O, NH ou S ; $R^1$ est un hydrogène, un alkyle, un alcényle, un alcynyle, un carbocyclyle, un hétérocyclyle, un aryle, ou un hétéroaryle ; chacun de m et n est indépendamment un entier de 5 à 2 000 ; et p est un entier de 1 à 10.

**2.** Formulation pharmaceutique selon la revendication 1, dans laquelle la nanoparticule est une micelle.

**3.** Formulation pharmaceutique selon la revendication 1, dans laquelle le bloc hydrophile a une masse moléculaire moyenne en nombre d'environ 500 Da à environ 20 kDa, ou le bloc hydrophile a une masse moléculaire moyenne en nombre d'environ 1 kDa à environ 8 kDa.

**4.** Formulation pharmaceutique selon la revendication 1, dans laquelle le bloc hydrophobe a une masse moléculaire moyenne en nombre d'environ 500 Da à environ 200 kDa, éventuellement dans laquelle le bloc hydrophobe a une

masse moléculaire moyenne en nombre d'environ 1 kDa à environ 50 kDa, ou une masse moléculaire moyenne en nombre d'environ 2 kDa à environ 20 kDa.

**5.** Formulation pharmaceutique selon l'une quelconque des revendications 1 à 4, dans laquelle la nanoparticule a un diamètre d'environ 10 nm à environ 1 000 nm, de préférence d'environ 30 nm à environ 500 nm, mieux encore d'environ 50 nm à environ 250 nm, dans laquelle le diamètre de la nanoparticule est mesuré par diffusion dynamique de la lumière.

**6.** Formulation pharmaceutique selon l'une quelconque des revendications 1 à 5, dans laquelle la surface de la nanoparticule comprend un fragment ciblant une glycoprotéine, éventuellement dans laquelle le fragment ciblant une glycoprotéine comprend un groupe acide boronique :

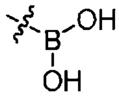

ou dans laquelle le fragment ciblant une glycoprotéine comprend un groupe maléimide :

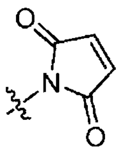

de préférence dans laquelle le fragment ciblant une glycoprotéine est couplé à l'extrémité distale (par exemple l'amine, l'hydroxyle ou le thiol) du bloc hydrophile, de préférence a la structure suivante :

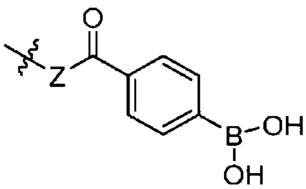

ou le fragment ciblant une glycoprotéine a la structure suivante :

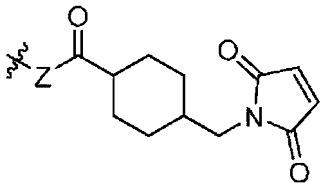

dans laquelle Z est O, NH ou S.

**7.** Formulation pharmaceutique selon l'une quelconque des revendications 1 à 6, dans laquelle la nanoparticule comprend en outre un agent thérapeutique, éventuellement dans laquelle l'agent thérapeutique est un agent antibactérien ou un agent anti-inflammatoire, de préférence l'agent antibactérien ou l'agent anti-inflammatoire est choisi parmi l'étabonate de lotéprednol, la dexaméthasone, et la ciprofloxacine.

**8.** Formulation pharmaceutique selon l'une quelconque des revendications 1 à 7, dans laquelle le polymère anionique est réticulé, de préférence dans laquelle le polymère anionique réticulé a une densité de réticulation d'environ 0,01 % à environ 2,0 %, éventuellement dans laquelle la densité de réticulation du polymère anionique est d'environ 0,05 % à environ 0,5 %, de préférence le polymère anionique comprend un motif répétitif choisi parmi

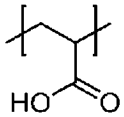 et 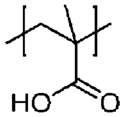

ou le polymère anionique comprend un motif de réticulation dérivé du divinylglycol, de préférence dans laquelle le polymère anionique est un polycarbophile, mieux encore le polycarbophile est un polycarbophile calcique, ou la matrice a un pourcentage en poids/volume (%w/v) de polycarbophile d'environ 0,01 % à environ 10,0 %, de préférence un pourcentage en poids/volume (%w/v) d'environ 0,1 % à environ 2,0 %.

**9.** Formulation pharmaceutique selon l'une quelconque des revendications 1 à 8, dans laquelle la matrice aqueuse comprenant le polymère ionique réticulé est un gel nondurcissant, éventuellement dans laquelle la matrice aqueuse comprend en outre du tyloxapol, ou la matrice aqueuse a un pourcentage en poids/volume (%w/v) de tyloxapol d'environ 0,01 % à environ 5,0 %, de préférence un pourcentage en poids/volume (%w/v) d'environ 0,1 % à environ 2,0 %, mieux encore un pourcentage en poids/volume (%w/v) d'environ 0,5 %, éventuellement dans laquelle la matrice aqueuse comprend en outre un chélatant, de préférence l'édétate disodique dihydraté (EDTA).

**10.** Formulation pharmaceutique selon l'une quelconque des revendications 1 à 9, dans laquelle la formulation pharmaceutique est formulée pour une administration par voie topique, éventuellement dans laquelle la formulation pharmaceutique est formulée pour une administration par voie topique sous la forme de gouttes ophtalmiques.

FIG. 1

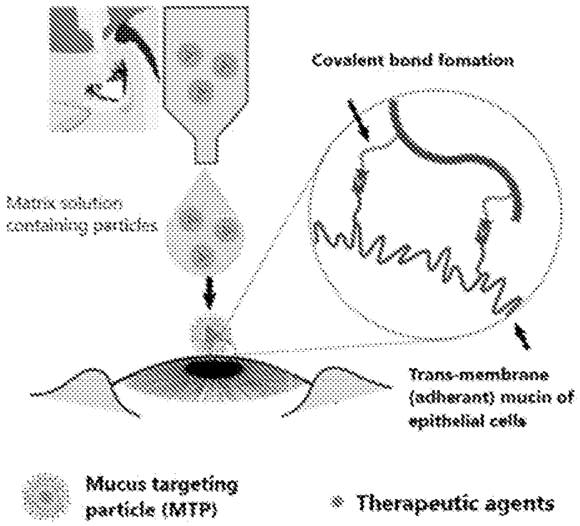
Covalent bond fomation
Matrix solution
containing particles
Trans-membrane
(adherant) mucin of
epithelial cells
Mucus targeting
particle (MTP)
Therapeutic agents

FIG. 2

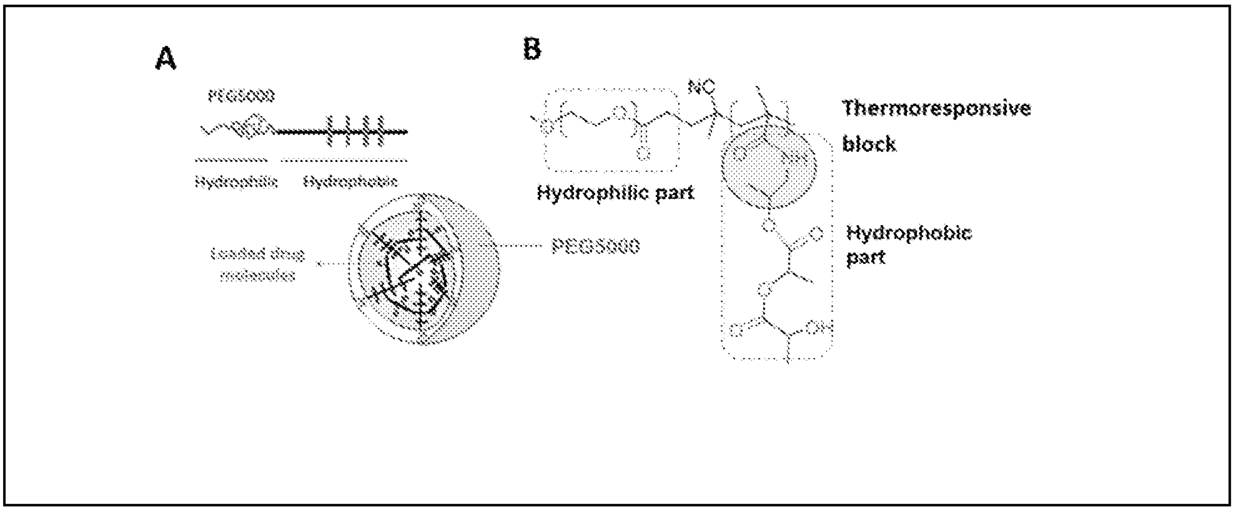
A
PEG5000
Hydrophilic
Hydrophobic
Loaded drug
molecules
PEG5000
B
Thermoresponsive
block
Hydrophilic part
Hydrophobic
part

**FIG. 3**

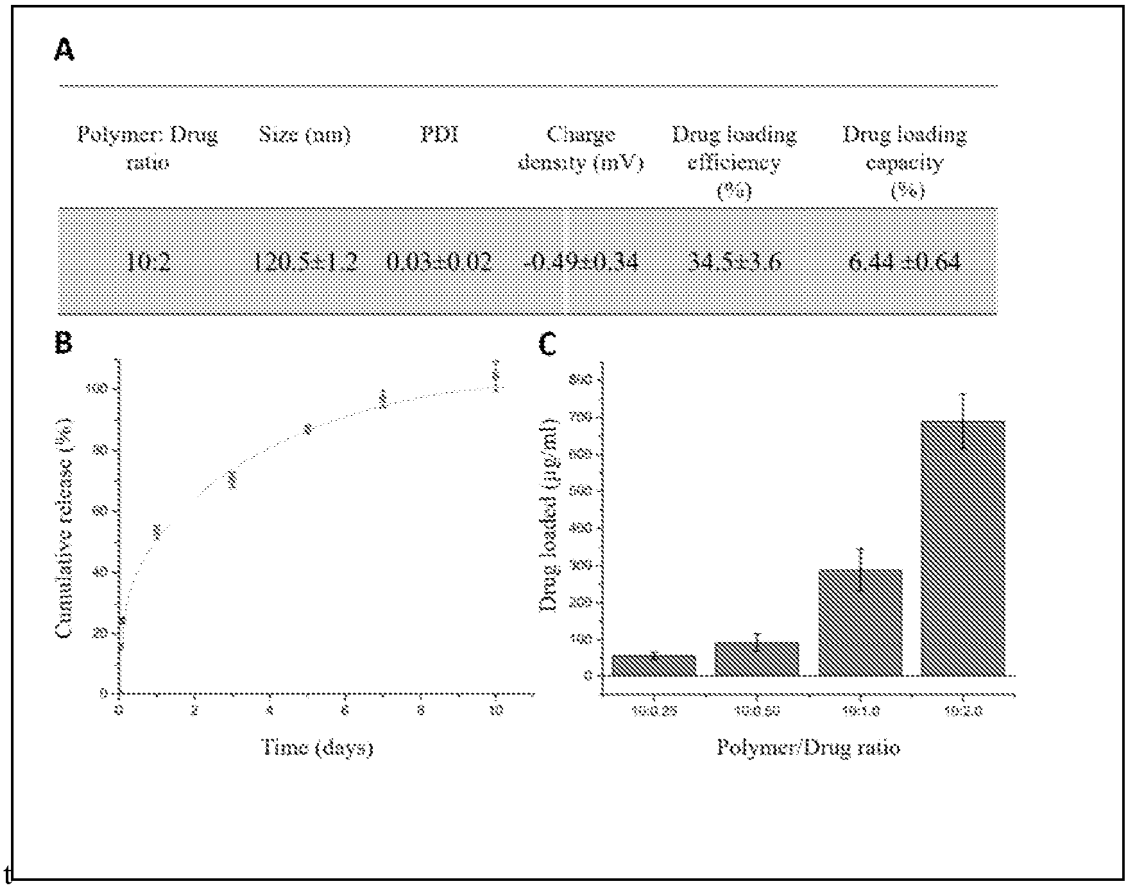

| Polymer: Drug ratio | Size (nm) | PDI | Charge density (mV) | Drug loading efficiency (%) | Drug loading capacity (%) |
|---|---|---|---|---|---|
| 10:2 | 120.5±1.2 | 0.03±0.02 | -0.49±0.34 | 34.5±3.6 | 6.44 ±0.64 |

FIG. 4

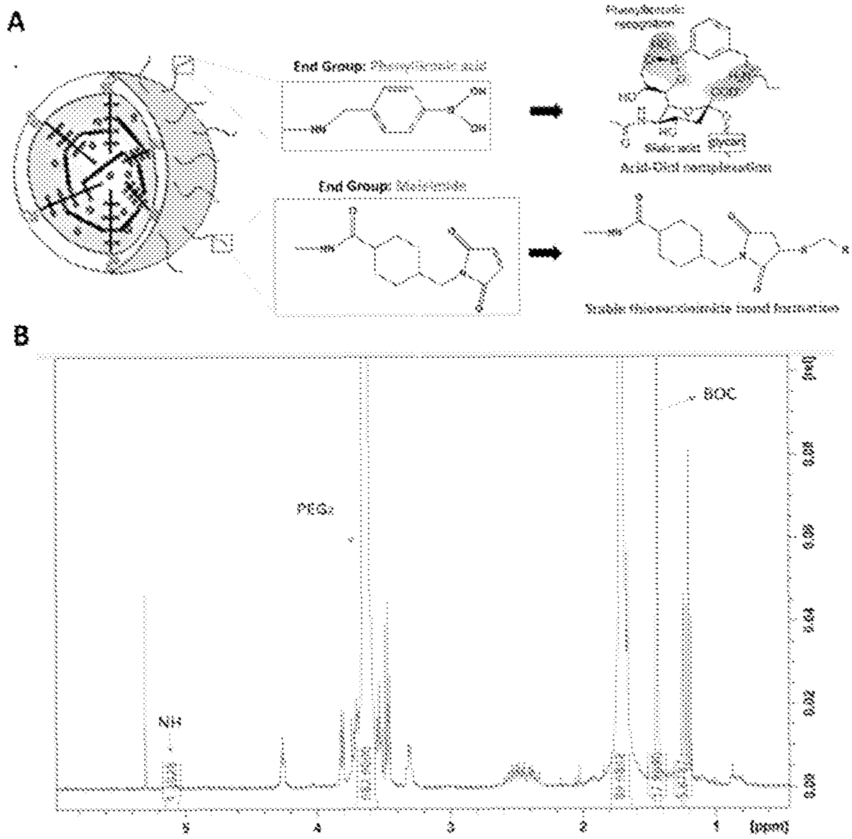
A
End Group:
End Group:
Acid-Diol complexation
Stable thiosuccinimide bond formation
B
BOC
PEGs
NH
5
4
3
2
1
[ppm]

FIG. 5

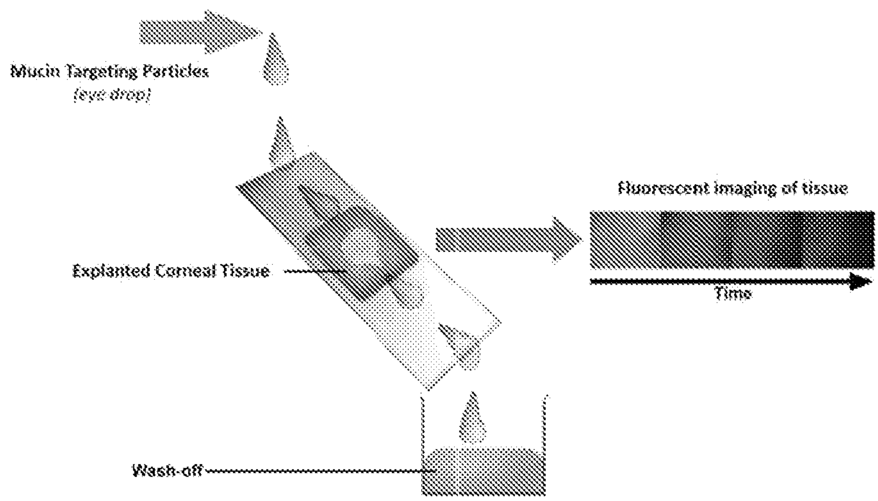
Mucin Targeting Particles
(eye drop)
Explanted Corneal Tissue
Fluorescent imaging of tissue
Time
Wash-off

FIG. 6

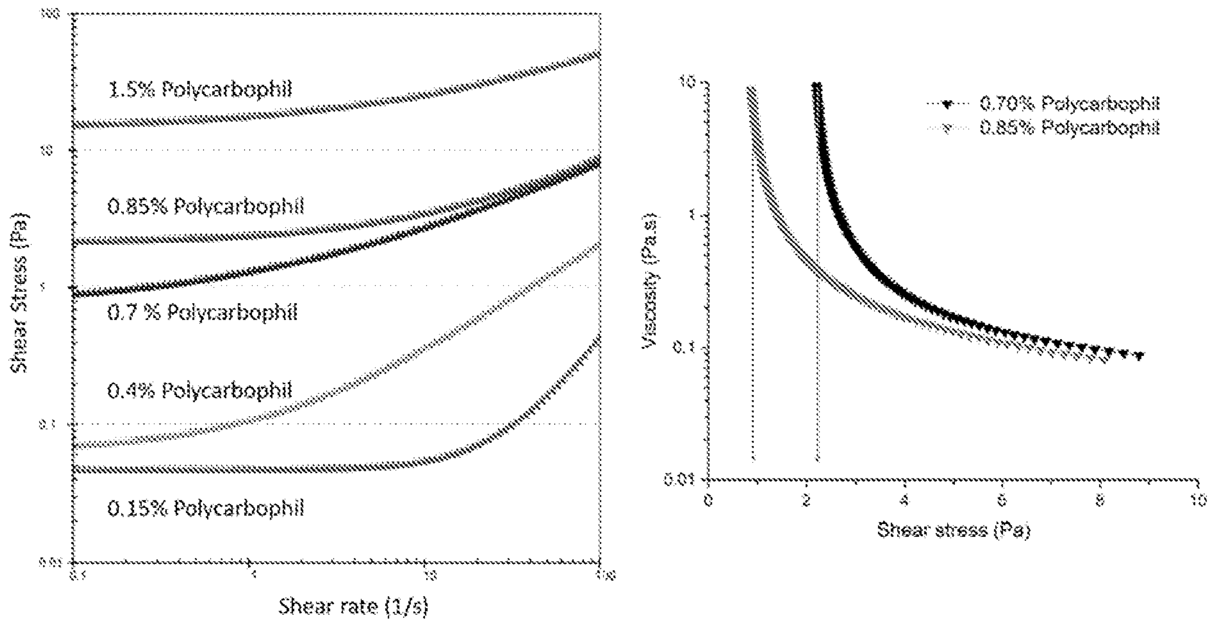
1.5% Polycarbophil
0.85% Polycarbophil
0.7 % Polycarbophil
0.4% Polycarbophil
0.15% Polycarbophil
Shear Stress (Pa)
Shear rate (1/s)
0.70% Polycarbophil
0.85% Polycarbophil
Viscosity (Pa.s)
Shear stress (Pa)
10
1
0.1
0.01
0
2
4
6
8
10

FIG. 7

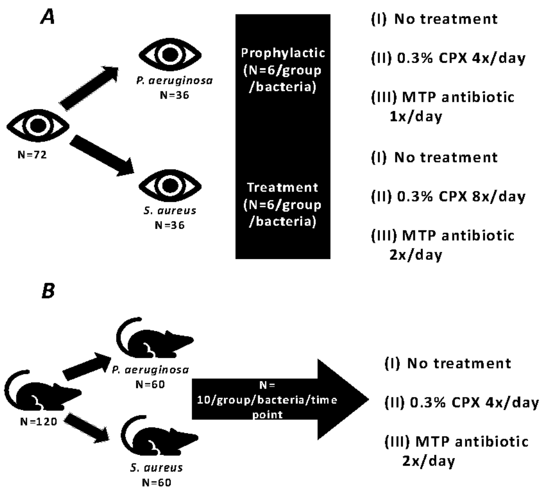
A
P. aeruginosa
N=36
N=72
S. aureus
N=36
Prophylactic (N=6/group /bacteria)
Treatment (N=6/group /bacteria)
(I) No treatment
(II) 0.3% CPX 4x/day
(III) MTP antibiotic 1x/day
(I) No treatment
(II) 0.3% CPX 8x/day
(III) MTP antibiotic 2x/day
B
P. aeruginosa
N=60
N=120
S. aureus
N=60
N= 10/group/bacteria/time point
(I) No treatment
(II) 0.3% CPX 4x/day
(III) MTP antibiotic 2x/day

FIG. 8

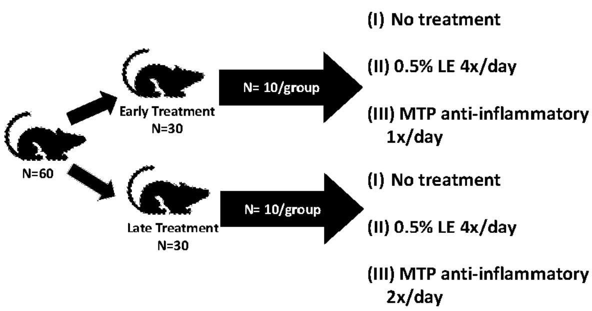
Early Treatment
N=30
N=60
Late Treatment
N=30
N= 10/group
N= 10/group
(I) No treatment
(II) 0.5% LE 4x/day
(III) MTP anti-inflammatory 1x/day
(I) No treatment
(II) 0.5% LE 4x/day
(III) MTP anti-inflammatory 2x/day

**FIG. 9**

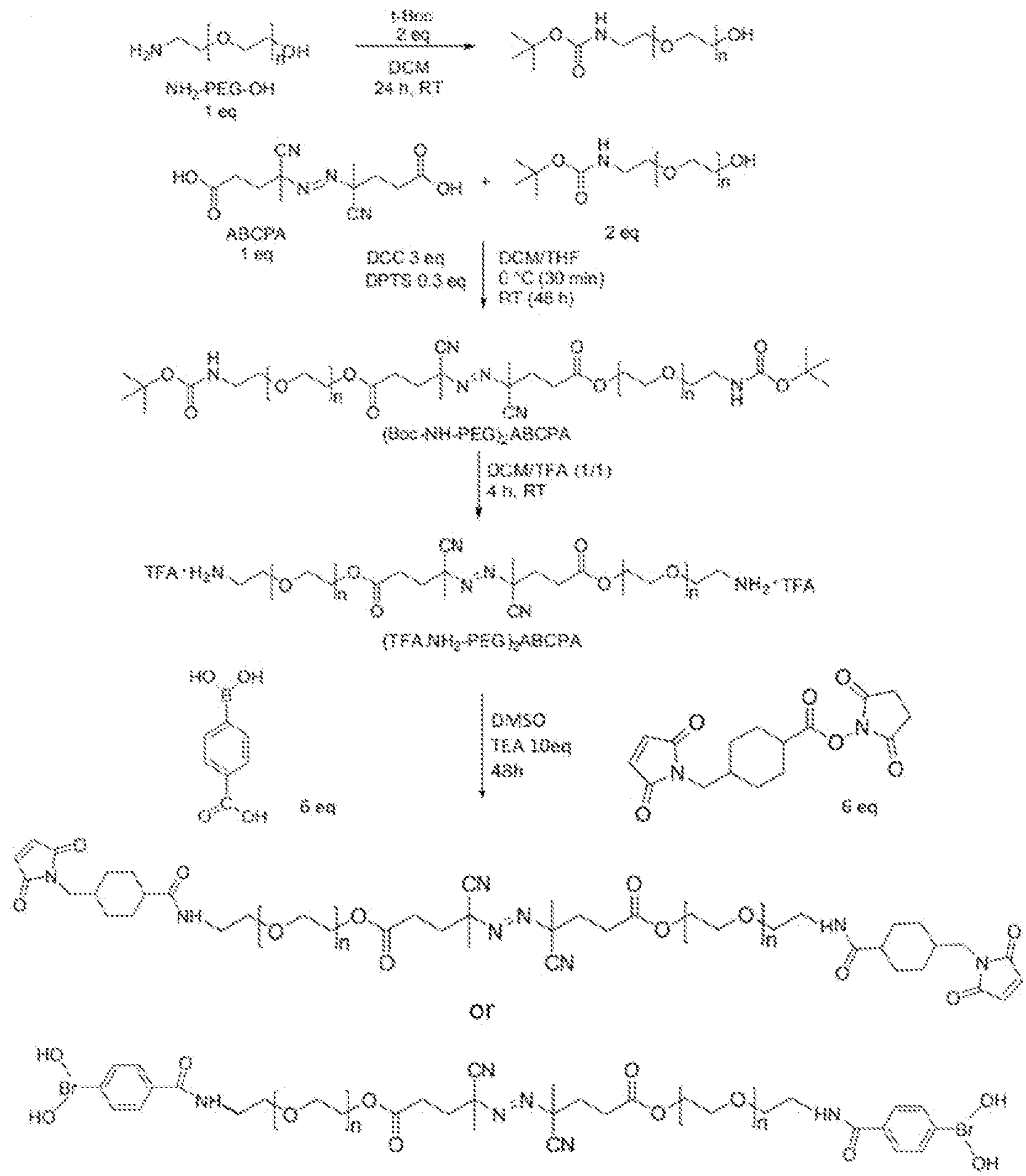
t-Boc
2 eq
DCM
24 h, RT
NH$_2$-PEG-OH
1 eq
ABCPA
1 eq
2 eq
DCC 3 eq
DPTS 0.3 eq
DCM/THF
0 °C (30 min)
RT (48 h)
(Boc-NH-PEG)$_2$-ABCPA
DCM/TFA (1/1)
4 h, RT
(TFA.NH$_2$-PEG)$_2$ABCPA
DMSO
TEA 10eq
48h
6 eq
6 eq

or

FIG. 10

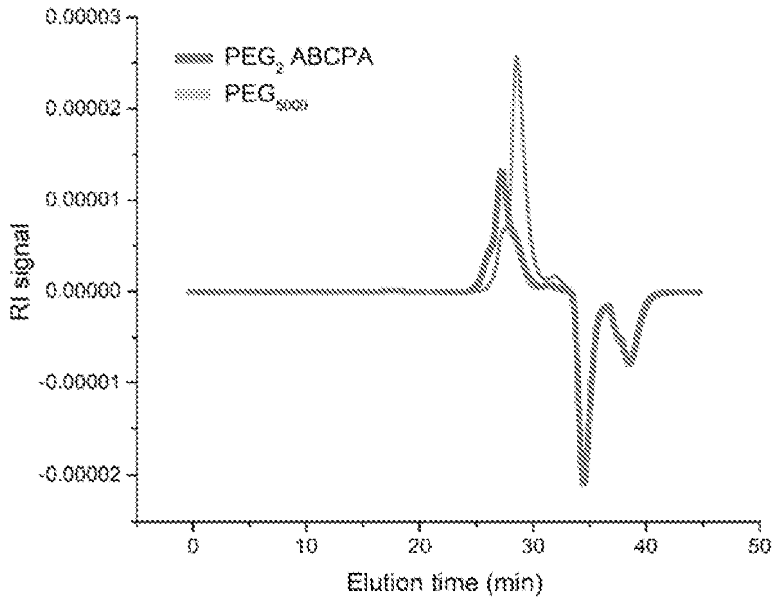


FIG. 11

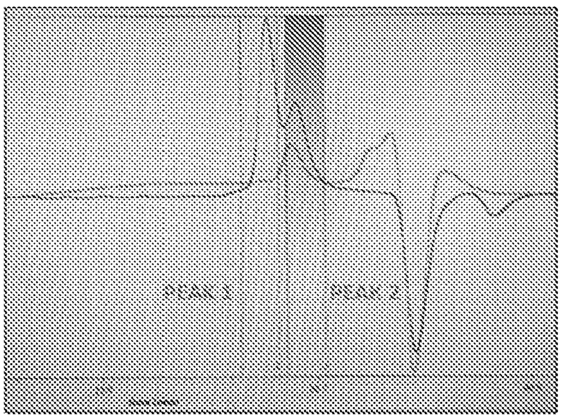


| | Mn (Da) | PDI | Retention time (min) |
|---|---|---|---|
| Boc-NH2-PEG | 3400 | 1.1 | 28.2 |
| (Boc-NH-PEG)2 -ABCPA | 6,260 | 1.24 | 26.5 |

FIG. 12

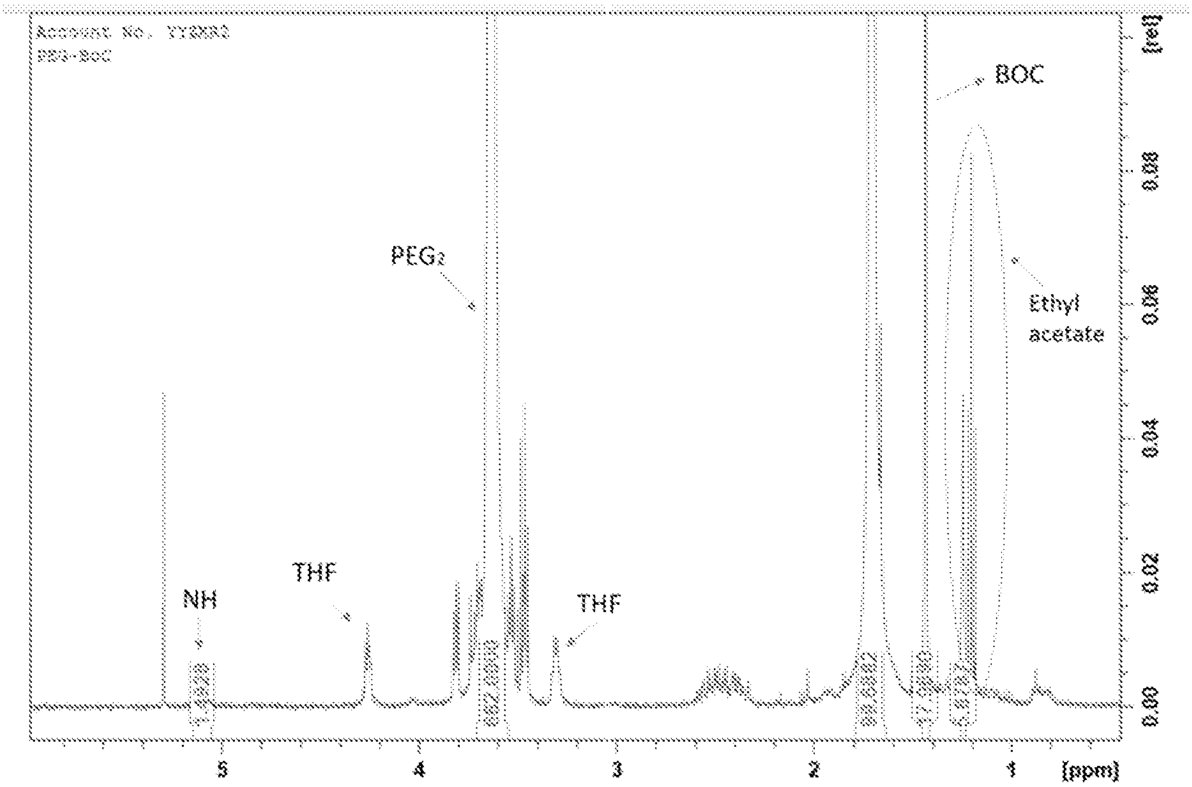
Account No. YY8RR2
PEG-BoC
BOC
PEG2
Ethyl
acetate
NH
THF
THF
5
4
3
2
1
[ppm]
0.08
0.06
0.04
0.02
0.00
[rel]

**FIG. 13**

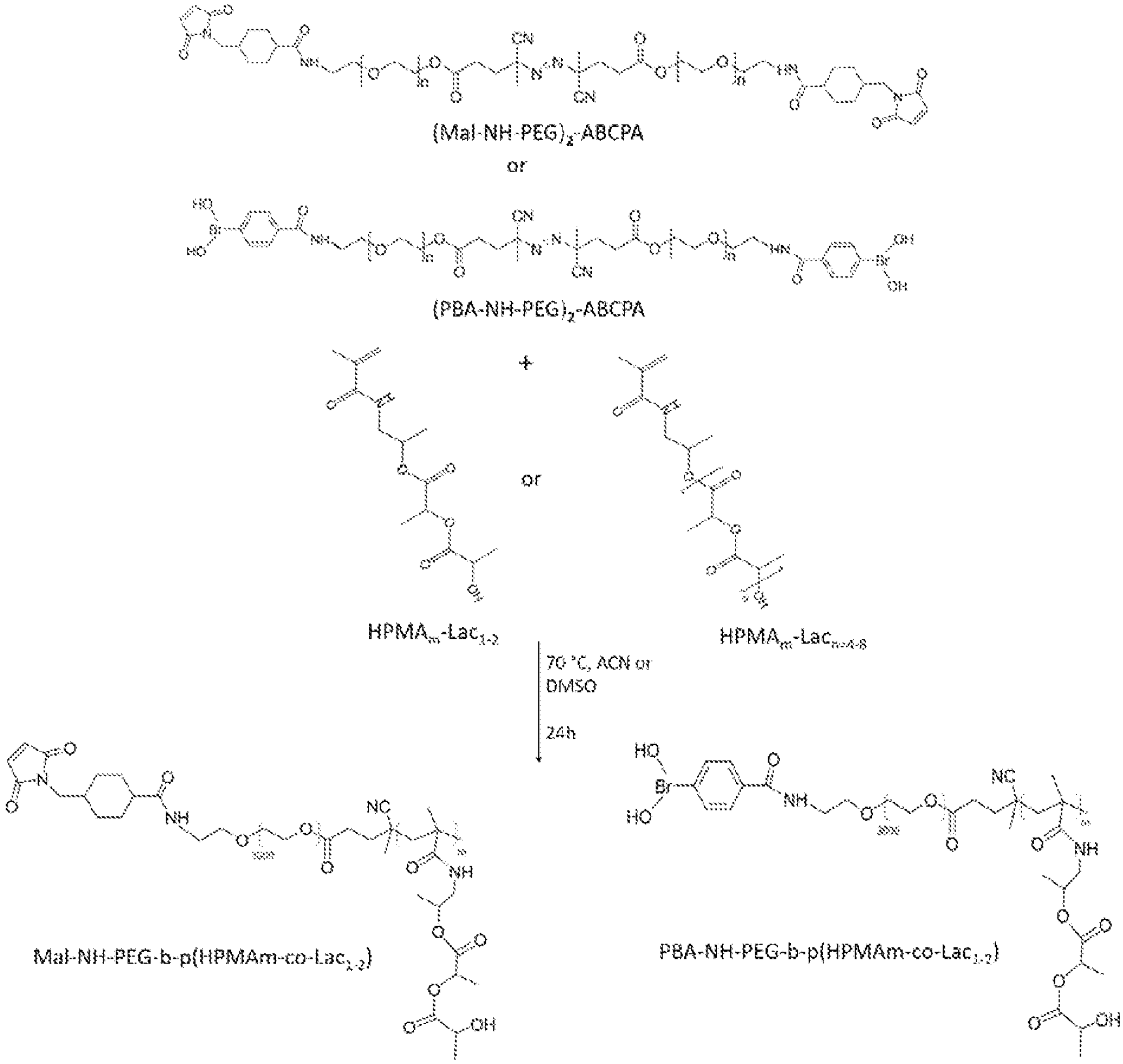


(Mal-NH-PEG)$_2$-ABCPA

or

(PBA-NH-PEG)$_2$-ABCPA

+

or

HPMA$_m$-Lac$_{1-2}$

HPMA$_m$-Lac$_{n=4-8}$

Mal-NH-PEG-b-p(HPMAm-co-Lac$_{1-2}$)

PBA-NH-PEG-b-p(HPMAm-co-Lac$_{1-2}$)

**FIG. 14**

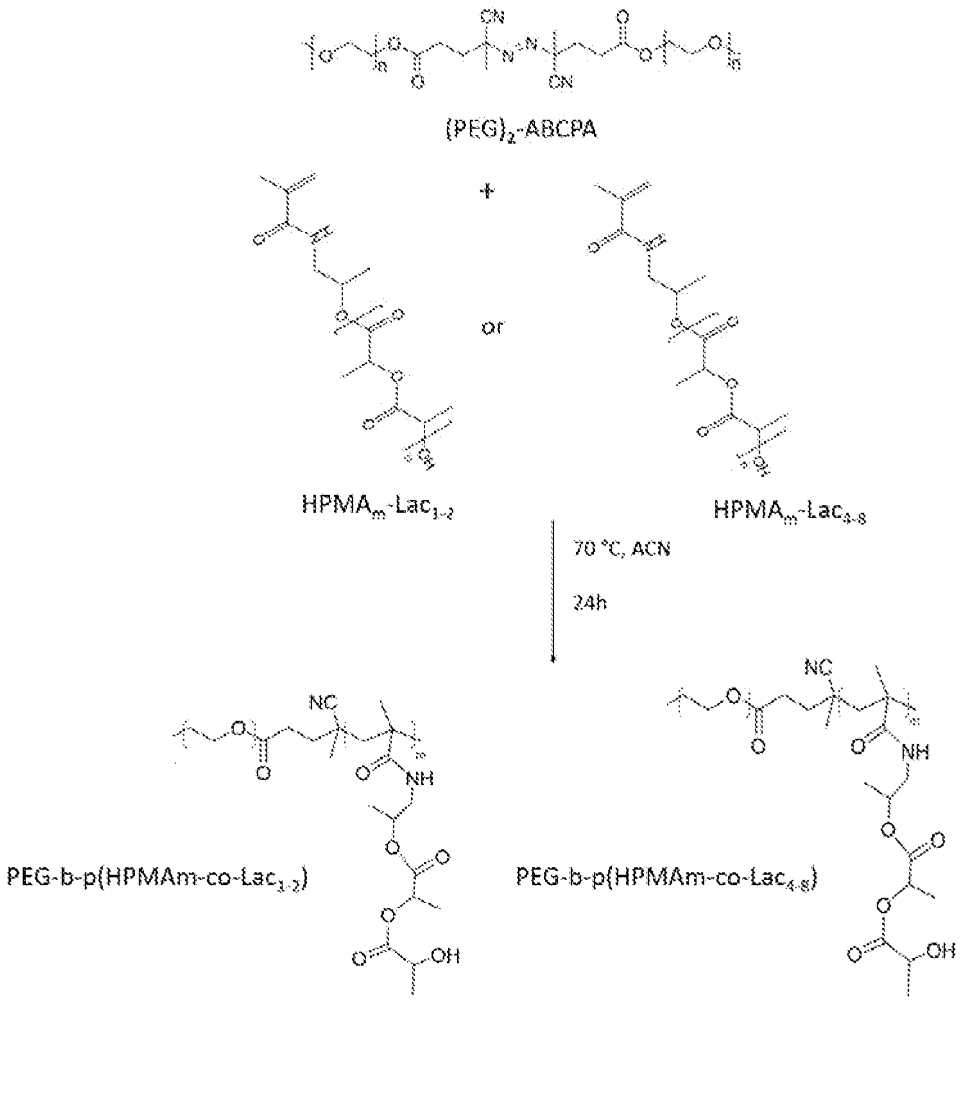


$(PEG)_2$-ABCPA

+

$HPMA_m$-$Lac_{1-2}$

or

$HPMA_m$-$Lac_{4-8}$

PEG-b-p(HPMAm-co-$Lac_{1-2}$)

PEG-b-p(HPMAm-co-$Lac_{4-8}$)

**FIG. 15**

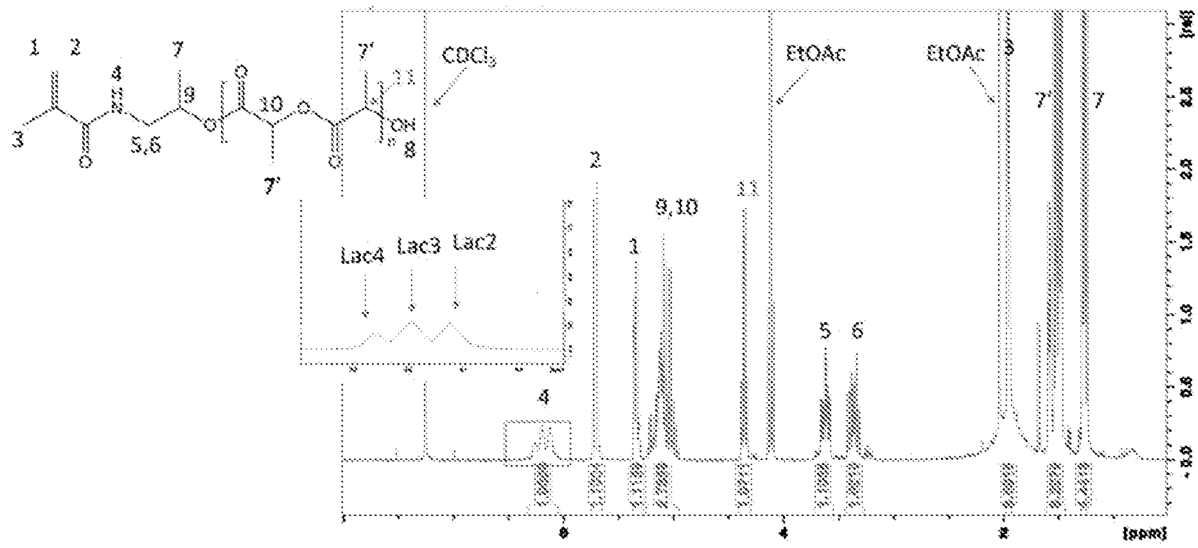
CDCl$_3$
EtOAc
EtOAc
Lac4
Lac3
Lac2
[ppm]

**FIG. 16**

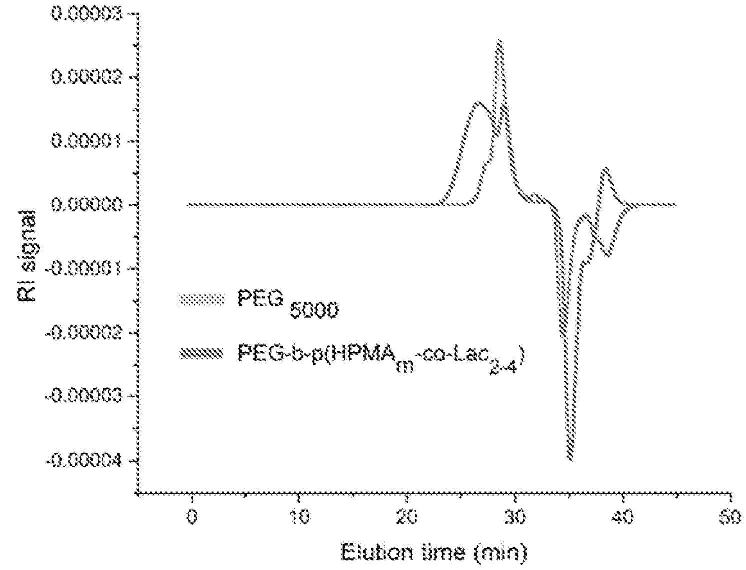
RI signal
PEG 5000
PEG-b-p(HPMAm-co-Lac2-4)
Elution time (min)

**FIG. 17**

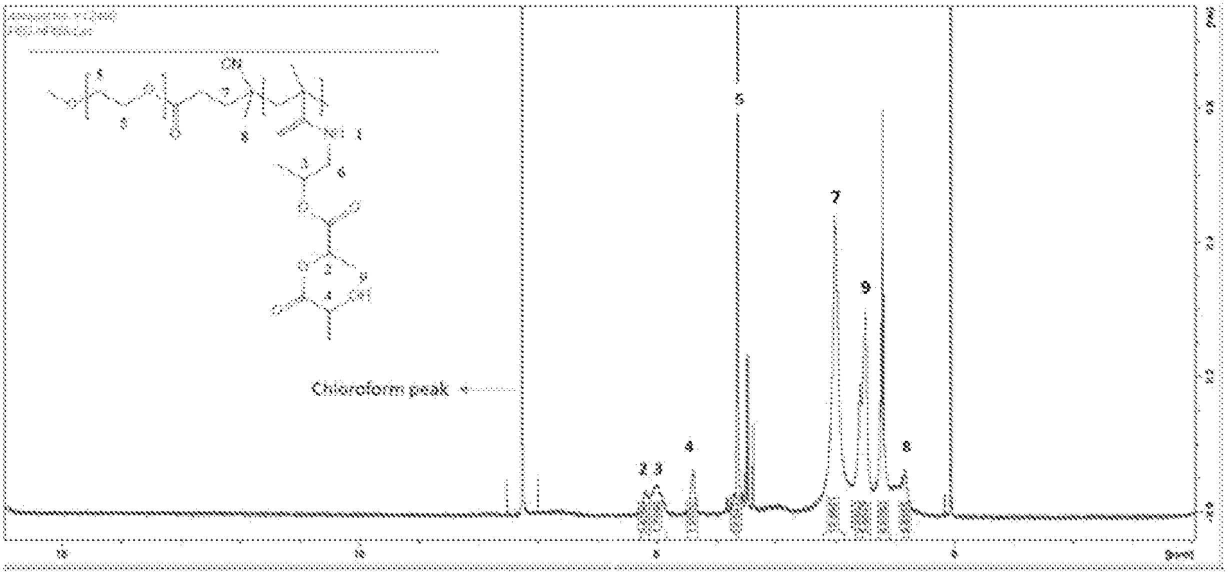

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 63123208
- WO 2013188979 A1
- US 6110973 A
- US 5763493 A
- US 5731000 A
- US 5541231 A
- US 5427798 A
- US 5358970 A
- US 4172896 A

### Non-patent literature cited in the description


- **ISSELBACHER et al.** Harrison's Principles of Internal Medicine. 1996, 1814-1882
- Principles of Neural Science. McGraw-Hill Medical, 2000
- **MOTULSKY**. Intuitive Biostatistics. Oxford University Press, Inc., 1995
- **LODISH et al.** Molecular Cell Biology. W. H. Freeman & Co., 2000
- **GRIFFITHS et al.** Introduction to Genetic Analysis. W. H. Freeman & Co., 1999
- **GILBERT et al.** Developmental Biology. Sinauer Associates, Inc., 2000
- The McGraw-Hill Dictionary of Chemical Terms. McGraw-Hill, 1985
- **GHATE D, E.H.** Ocular drug delivery. *Expert Opinion on Drug Delivery*, 2006, vol. 3, 275-87
- **SEARS ML**. Pharmacology of the Eye. Springer-Verlag, 1984
- **BUCOLO, C.** ; **F. DRAGO** ; **S. SALOMONE**. Ocular drug delivery: a clue from nanotechnology. *Front Pharmacol*, 2012, vol. 3, 188
- **V.H.L. LEE, J.R.R.** Review: topical ocular drug delivery: recent developments and future challenges. *J. Ocul. Pharmacol.*, 1986, vol. 2, 67-108
- **A. TOPALKARA, C.G.L.** ; **D.S. ARICI** ; **M.K. ARICI**. Adverse effects of topical antiglaucoma drugs on the ocular surface. *Clin. Exp. Ophthalmol*, 2000, vol. 28, 113-117
- **NAGARWAL RC, K.S.** ; **SINGH PN** ; **MAITI P** ; **PANDIT JK**. Polymeric nanoparticulate system: a potential approach for ocular drug delivery. *Journal of Controlled Release*, 2009, vol. 136 (1), 2-13
- **MARTIN J COFFEY, H.H.D.** ; **STEPHEN S LANE**. Development of a non-settling gel formulation of 0.5% loteprednol etabonate for anti-inflammatory use as an ophthalmic drop. *Clin Ophthalmol.*, 2013, vol. 7, 299-312
- **LAI SK, W.Y.** ; **HANES J.** Mucus-penetrating nanoparticles for drug and gene delivery to mucosal tissues. *Adv Drug Deliv Rev*, 2009, vol. 61 (2), 158-71
- **HU Q, R.C.** ; **VAN GAAL E** ; **BRUNDEL P** ; **KOSTKOVA H** ; **ETRYCH T** ; **WEBER B** ; **BARZ M** ; **KIESSLING F** ; **PRAKASH J** ; **STORM G**. Tailoring the physicochemical properties of corecrosslinked polymeric micelles for pharmaceutical applications. *J Control Release*, 2016, vol. 244, 314-325
- **RIJCKEN, C.J.F.** *Tunable and degradable polymeric micelles for drug delivery: from synthesis to feasibility invivo*, 2007
- **FUJII, N.K.A.S.** Recent Advances in Ocular Drug Delivery Systems. *Polymers*, 2011, vol. 3, 193-221